# EUROPEAN PATENT SPECIFICATION

(11) **EP 2 414 548 B1**
(45) Date of publication and mention of the grant of the patent: **21.10.2015**
(21) Application number: 10762102.1
(22) Date of filing: 23.03.2010
(51) Int. Cl.: C12Q 1/68, C12N 15/11, C40B 50/06

(54) **GENE EXPRESSION ANALYSIS IN SINGLE CELLS**
GENEXPRESSIONSANALYSE IN EINZELZELLEN
ANALYSE DE L'EXPRESSION GÉNÉTIQUE DANS DES CELLULES INDIVIDUELLES

(30) Priority: 30.03.2009 US 164759 P
(43) Date of publication of application: 08.02.2012
(73) Proprietor: Illumina, Inc., San Diego, CA 92122 (US)
(72) Inventor: LINNARSSON, Sten, San Diego, CA 92121 (US)
(74) Representative: Leissler-Gerstl, Gabriele
(86) International application number: PCT/US2010/028361
(87) International publication number: WO 2010/117620

(56) References cited:
- US-A1- 2007 105 090
- US-A1- 2007 117 121
- US-B1- 6 197 554
- G. MIKE MAKRIGIORGOS ET AL: "A PCR-based amplification method retaining the quantitative difference between two complex genomes", NATURE BIOTECHNOLOGY, vol. 20, no. 9, 1 September 2002 (2002-09-01), pages 936-939, XP55023044, ISSN: 1087-0156, DOI: 10.1038/nbt724
- MEGAN L MCCLOSKEY ET AL: "Encoding PCR Products with Batch-stamps and Barcodes", BIOCHEMICAL GENETICS, KLUWER ACADEMIC PUBLISHERS-PLENUM PUBLISHERS, NE, vol. 45, no. 11-12, 23 October 2007 (2007-10-23), pages 761-767, XP019548696, ISSN: 1573-4927, DOI: 10.1007/S10528-007-9114-X
- TANG FUCHOU ET AL: "RNA-Seq analysis to capture the transcriptome landscape of a single cell", NATURE PROTOCOLS, NATURE PUBLISHING GROUP, GB, vol. 5, no. 3, 1 March 2010 (2010-03-01), pages 516-535, XP009162232, ISSN: 1750-2799
- ISLAM SAIFUL ET AL: "Highly multiplexed and strand-specific single-cell RNA 5' end sequencing", 1 May 2012 (2012-05-01), NATURE PROTOCOLS, NATURE PUBLISHING GROUP, GB, PAGE(S) 813 - 828, XP009162220, ISSN: 1750-2799 * the whole document *
- ISLAM SAIFUL ET AL: "Characterization of the single-cell transcriptional landscape by highly multiplex RNA-seq", GENOME RESEARCH, vol. 21, no. 7, July 2011 (2011-07), pages 1160-1167, XP002682367,
- KAZUKI KURIMOTO ET AL.: 'An improved single-cell cDNA amplification method for efficient high-density oligonucleotide microarray analysis.' NUCLEIC ACIDS RESEARCH vol. 34, no. 5, E42, 2006, pages 1 - 17, XP008151612
- JAE-HYEONG KO ET AL.: 'RNA-conjugated template-switching RT-PCR method for generating an Escherichia coli cDNA library for small RNAs.' JOURNAL OF MICROBIOLOGICAL METHODS. vol. 64, no. 3, 2006, pages 297 - 304, XP027926770
- KAZUKI KURIMOT ET AL.: 'Global single-cell cDNA amplification to provide a template for representative high-density oligonucleotide microarray analysis.' NATURE PROTOCOLS. vol. 2, no. 3, 2007, pages 739 - 752, XP008151777
- BRADY G ET AL: "CONSTRUCTION OF CDNA LIBRARIES FORM SINGLE CELLS", METHODS IN ENZYMOLOGY, ACADEMIC PRESS, US, vol. 225, 1 January 1993 (1993-01-01), pages 611-623, XP008023533, ISSN: 0076-6879, DOI: 10.1016/0076-6879(93)25039-5
- MAEDA NORIHIRO ET AL: "Development of a DNA barcode tagging method for monitoring dynamic changes in gene expression by using an ultra high-throughput sequencer", July 2008 (2008-07), BIOTECHNIQUES, VOL. 45, NR. 1, PAGE(S) 95-97 ISSN: 0736-6205
- Nicole Cloonan ET AL: "Stem cell transcriptome profiling via massive-scale mRNA sequencing", Nature Methods, vol. 5, no. 7, 30 May 2008 (2008-05-30), pages 613-619, XP55127676, ISSN: 1548-7091, DOI: 10.1038/nmeth.1223

## Description

### FIELD OF THE INVENTION

The present invention relates to the analysis of gene expression in single cells. In particular, the invention relates to a method for preparing a cDNA library from a plurality of single cells, and to a cDNA library produced by this method. The cDNA libraries prepared by the method of the invention are suitable for analysis of gene expression by sequencing.

### BACKGROUND OF THE INVENTION

The determination of the mRNA content of a cell or tissue (i.e. "gene expression profiling") provides a method for the functional analysis of normal and diseased tissues and organs. For example, gene expression profiling can be used in the study of embryogenesis; for the characterization of primary tumor samples; for the analysis of biopsies from diseased and normal tissue in, for example, psoriasis; for the comparative analysis of cell types from different species to delineate the evolution of development; as an assay system for diagnostics; as a quality control system in cell replacement therapy (i.e. to ensure that a culture of cells is sufficiently pure, and the cells are correctly differentiated); and as an *in vitro* tool to measure the effect of a transfected gene or siRNA on downstream targets in spite of less than 100% transfection efficiency.

Gene expression profiling is usually performed by isolating mRNA from tissue samples and subjecting this mRNA to microarray hybridization. However, such methods only allow previously known genes to be analyzed, and cannot be used to analyze alternative splicing, promoters and polyadenylation signals.

Therefore, direct sequencing of the all, or parts, of the mRNA content of a tissue is being increasingly used (Cloonan et al., Nat Methods 5(7):613-9 (2008)). However, current methods of analyzing the mRNA content of cells by direct sequencing rely on analyzing bulk mRNA obtained from tissue samples typically containing millions of cells. This means that much of the functional information present in single cells is lost or blurred when gene expression is analyzed in bulk mRNA. In addition, dynamic processes, such as the cell cycle, cannot be observed in population averages. Similarly, distinct cell types in a complex tissue (e.g. the brain) can only be studied if cells are analyzed individually.

Gene expression in single cells has previously been analyzed using a variety of methods (see, for example, Brail et al., Mutat Res 406(2-4):45-54 (1999) ; Levsky et al., Science 297(5582):836-40 (2002); Bengtsson et al. Genome Res 15(10):1388-92 (2005); Esumi et al., Nat Genet 37(2):171-6 (2005). In particular, single cell gene expression in neural cells has been studied by microarray analysis (see Esumi et al. Neurosci Res 60(4):439-51 (2008)). However, these methods require that each single cell is analyzed individually and treated separately during the entire procedure, which is time-consuming and expensive. In addition, the preparation and amplification of samples from single cells independently potentially introduces cell-to-cell variation. Furthermore, as the cDNA of each cell must be amplified to an amount that can be reasonably handled for the subsequent analysis, there is potential amplification bias. For example, a single cell contains about 0.3 pg of mRNA, and at least a 300 ng is commonly needed for subsequent analysis by sequencing. Therefore, an amplification of at least a million-fold is required.

Additionally, microarrays have two major shortcomings: they are linked to known genes, and they have limited sensitivity and dynamic range. RNA sequencing (RNA-Seq) overcomes these problems by sequencing RNA directly (Ozsolak et al., Nature 461:814-818 (2009)) or after reverse-transcription to cDNA (Cloonan et al., Nat. Methods 5:613-619 (2008); Mortazavi et al., Nat. Methods 5:621-628 (2008); Wang et al., Nature 456:470-476 (2008)). Sequence reads are mapped to the genome to reveal sites of transcription, and quantitation is based simply on hit counts, with great sensitivity and nearly unlimited dynamic range.

Tissues are rarely homogeneous, however, and therefore any expression profile based on a tissue sample, biopsy or cell culture will confound the true expression profiles of its constituent cells. One way of getting around this problem would be to analyze single cells instead of cell populations, and indeed single-cell methods have been developed for both microarrays (Esumi et al., Neurosci. Res. 60:439-451 (2008) and Kurimoto et al., Nucleic Acids Res. 34:42 (2006)). These methods are suitable for the analysis of small numbers of single cells, and in particular may be used to study cells that are difficult to obtain in large numbers, such as oocytes and the cells of the early embryo. Cells may be isolated for example by laser capture microdissection, or by microcapillary, and marker genes may be used to locate cells of interest. However, single-cell transcriptomics must confront two great challenges. First, markers suitable for the prospective isolation of defined cell populations are not available for every cell type, reflecting the fact that few cell types are clearly defined in molecular terms. Second, transcript abundances vary greatly from cell to cell. For example, (β-Actin (Actb) mRNA content varies more than three orders of magnitude between pancreatic islets cells (Bengtsson et al., Genome Res. 15:1388-1392 (2005)). Similar results have been reported, using a variety of detection methods, for RNA polymerase II (Raj et al., PLoS Biol 4:309 (2006)), GAPDH (Lagunavicius et al., RNA 15:765-771 (2009) and Warren et al., Proc. Natl. Acad. Sci. U.S.A. 103:17807-17812 (2006)), PU.1 (Warren et al., *supra*), and TBP, B2M, SDHA and EE1FG mRNAs (Taniguchi et al., Nat Methods 6:503-506 (2009)), and at present seems to be a common feature of the transcriptome.

Most of the variation may be intrinsic, caused by burst-like stochastic activation of transcription, where brief episodes of mRNA synthesis lasting a few minutes are separated by periods of transcriptional silence of similar duration (Chubb et al., Curr Biol 16:1018-1025 (2006)). Each burst would give rise to a dense population of mRNA in the nucleus, which is then exported to the cytoplasm and rapidly decays. As a consequence, a random sample of cells would show great variation in their content of particular mRNAs, ranging from those cells that have just undergone a burst, to those that have nearly completely degraded their mRNA; this has been directly observed for RNA polymerase II transcription *in situ* using a fluorescent probe targeting the 52-copy repeat in that gene (Raj et al., PLoS Biol 4:309 (2006)).

In summary, there are often no suitable cell-surface markers to use in isolating single cells for study, and even when there are, a small number of single cells is not sufficient to capture the range of natural variation in gene expression. The present invention aims to overcome, or reduce, these problems by providing a method of preparing cDNA libraries which can be used to analyze gene expression in a plurality of single cells.

### SUMMARY OF THE INVENTION

The invention provides method for preparing a cDNA library from a plurality of single cells. In one aspect, the method includes the steps of releasing mRNA from each single cell to provide a plurality of individual mRNA samples, synthesizing a first strand of cDNA from the mRNA in each individual mRNA sample and incorporating a tag into the cDNA to provide a plurality of tagged cDNA samples, pooling the tagged cDNA samples and amplifying the pooled cDNA samples to generate a cDNA library having double-stranded cDNA. The invention also provides a cDNA library produced by the methods described herein. The invention further provides methods for analyzing gene expression in a plurality of cells by preparing a cDNA library as described herein and sequencing the library.

### BRIEF DESCRIPTION OF THE DRAWINGS

The figures are intended to illustrate broad concepts of the invention by reference to representative examples for ease of discussion. They are not intended to limit the scope of the invention by showing one out of several alternate embodiments or by showing or omitting optional features of the invention.

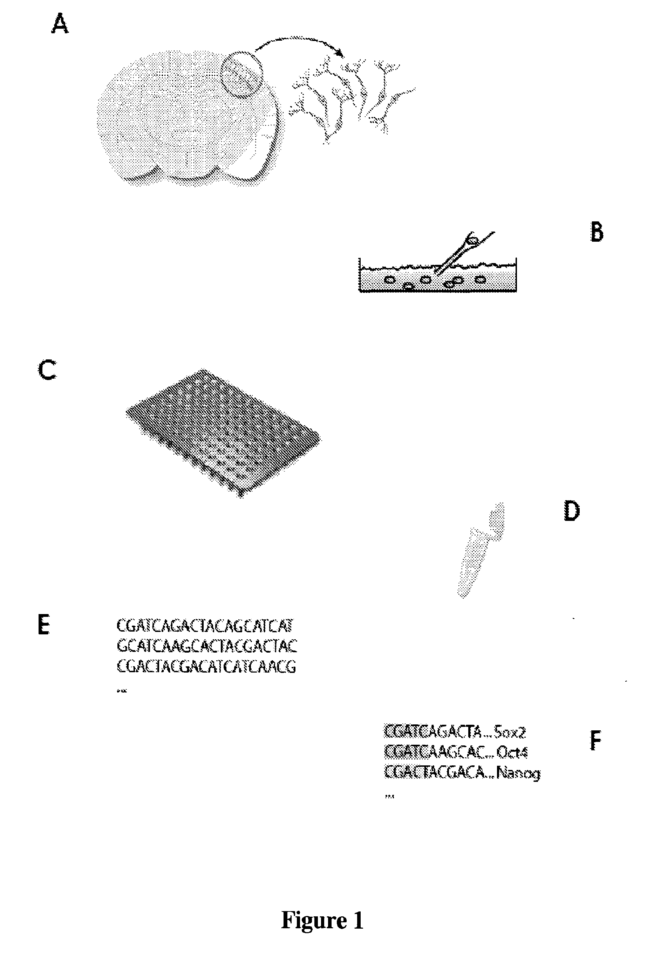

**Figure 1****,** panels A-F, show an overview of a method of analyzing gene expression in a plurality of single cells. (A) the tissue of interest is dissected; (B) a plurality of single cells are selected; (C) single cells are placed in separate wells of a 96-well plate and lysed; tagged reverse transcription is performed on each sample to produce cDNA; (D) cDNA samples are pooled and amplified; (E) sequencing is performed to obtain 100 million reads; and (F) identification of expressed genes and identification of cells from which they originated.

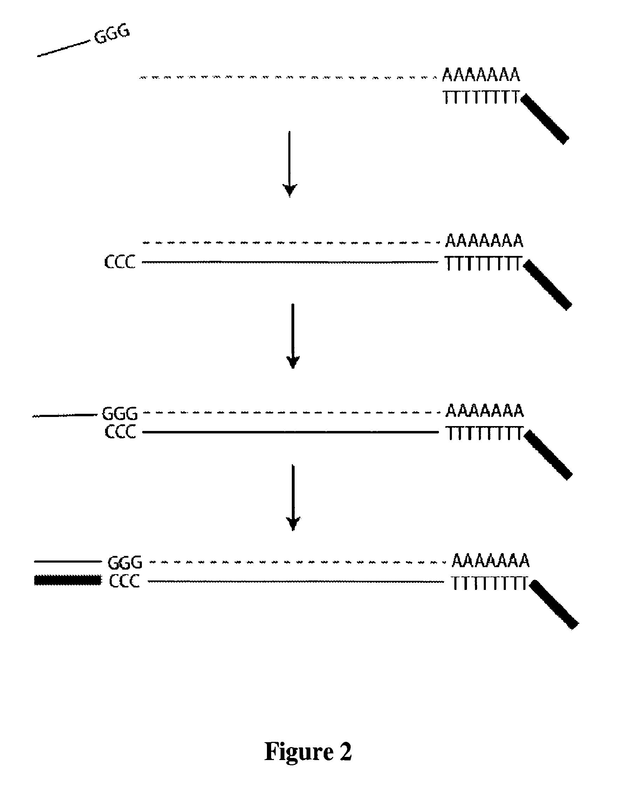

**Figure 2** shows the synthesis of cDNA by template switching.

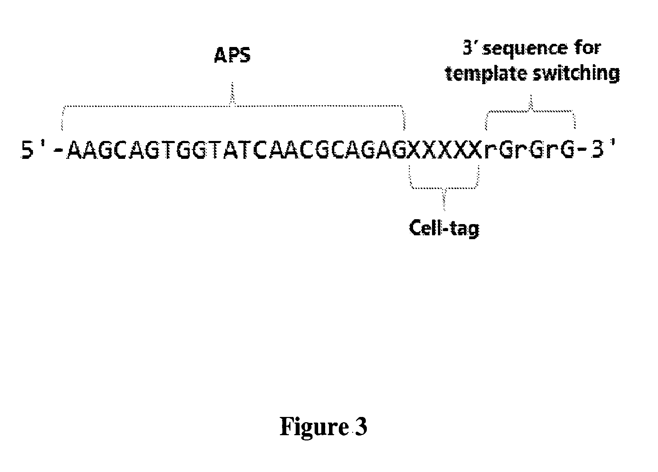

**Figure 3** shows an example of a template switching oligonucleotide comprising a 5' amplification primer sequence (APS), a cell tag and a 3' sequence for template switching.

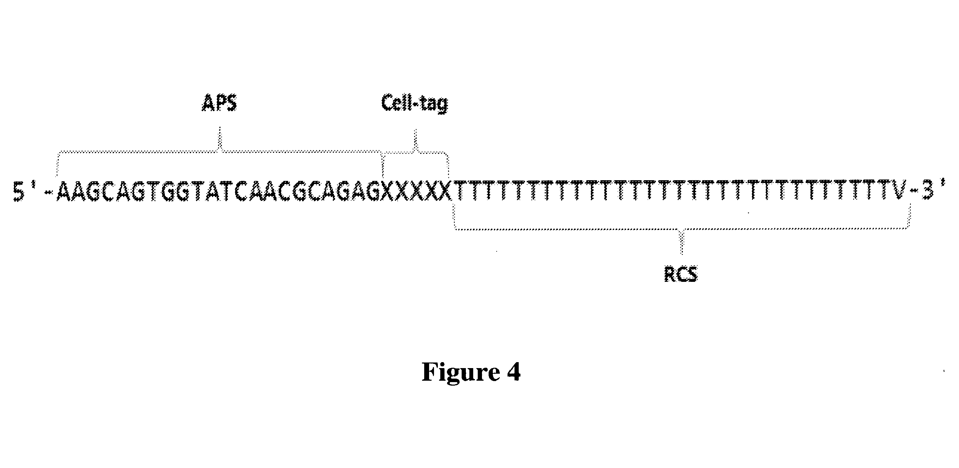

**Figure 4** shows an example of a cDNA synthesis primer (CDS) comprising a 5' amplification primer sequence (APS), a cell tag and a 3' RNA complementary sequence (RCS).

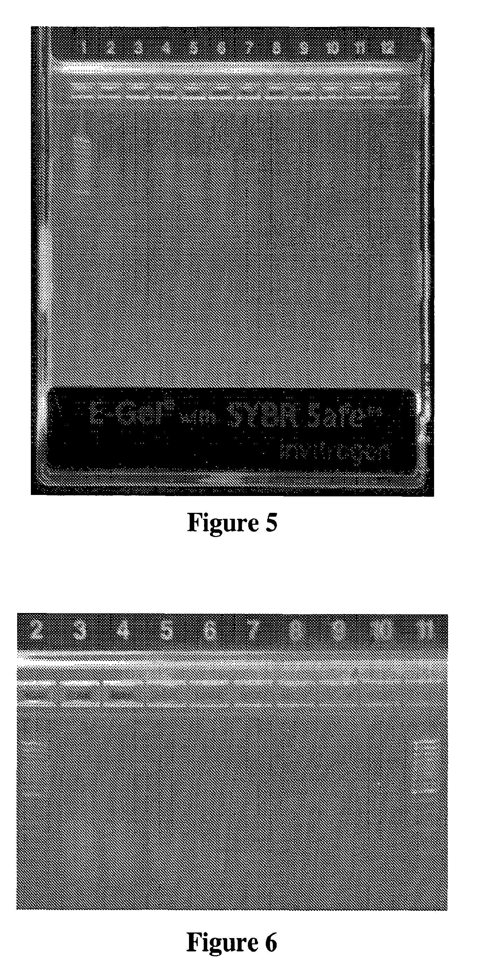

**Figure 5** shows the visualization of cDNA samples L001 and L002 following full-length cDNA amplification by PCR. Lane 1: 100 bp marker ladder; lanes 2-3: 25 cycles; lanes 4-5: 30 cycles; lanes 6-7: 35 cycles. Even lanes contain sample L001 and odd lanes contain sample L002.
**Figure 6** shows a dilution series from a test PCR using sample L001 (lanes 3-10). Lanes 2 and 11 contain a 100 bp ladder as a size marker.

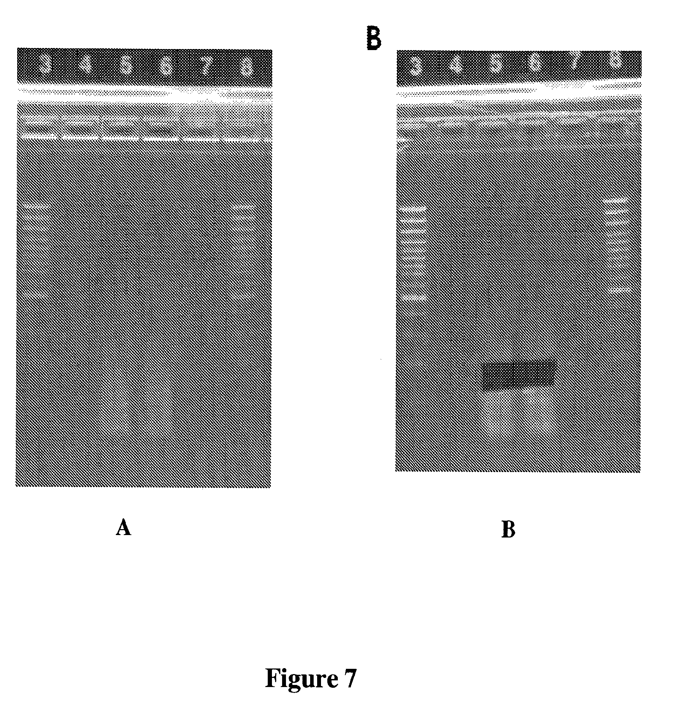

**Figure 7****,** panels A and B, show the gel electrophoresis separation and isolation of cDNA libraries. Panel A shows a cDNA library following final amplification by PCR (16 cycles) (lanes 5 and 6). Panel B shows the 125-200 bp region has been excised. Lanes 3 and 8 contain 100 bp ladder as a size marker.

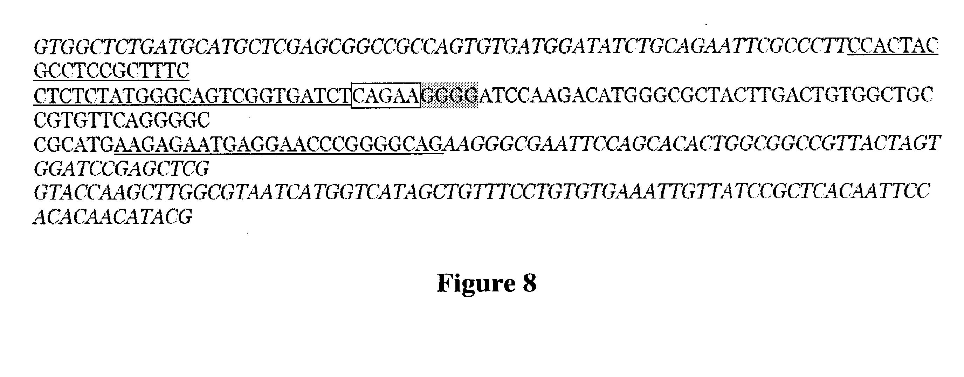

**Figure 8** shows an example of a sequenced cDNA molecule from a tagged cDNA library. The primer sequences for SOLiD sequencing (P1 and P2) are underlined. The cell-specific tag is boxed. The 2-5 Gs from the template-switching mechanism are shaded by a grey box. Sequence from the TOPO cloning vector is shown in italics. The insert in this case is tubulin beta 2c.

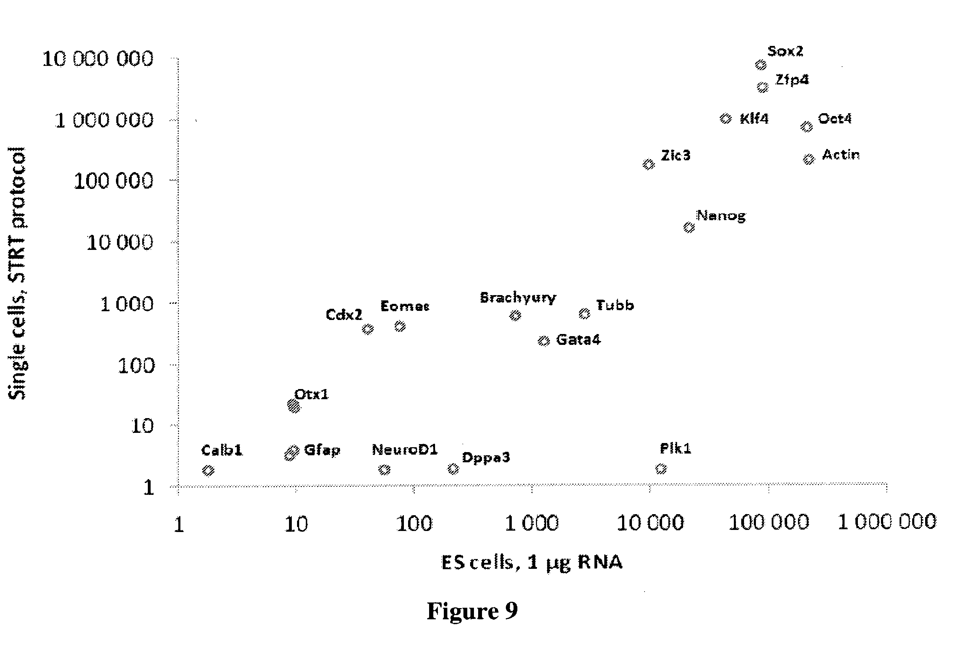

**Figure 9** shows a graphical representation of results from a quantitative real-time PCR comparing bulk cDNA (horizontal axis) *versus* 96-cell tagged cDNA (vertical axis). Each circle represents a PCR primer pair directed against the indicated genes. The units are arbitrary and are derived from the cycles-to-threshold value, C_{T}.

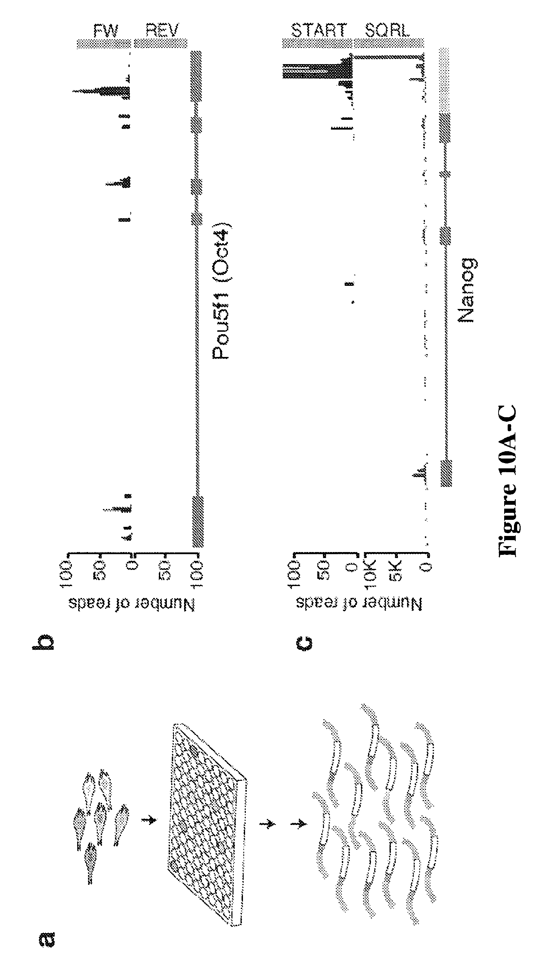

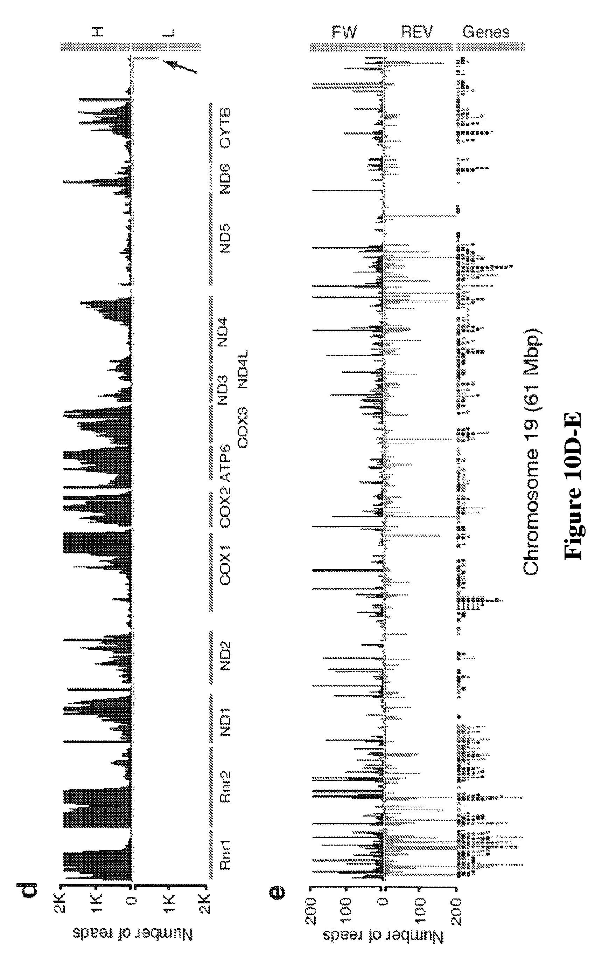

**Figure 10****,** panels A-E, show an overview of the single-cell tagged reverse transcription (STRT) method and exemplary results. (A) Overview of the method, illustrating how single cells were tracked. Well-specific (and hence cell-specific) barcodes were incorporated during cDNA synthesis, resulting in a library where each molecule carried a barcode identifying the cell of origin. (B) Example of reads mapped to both strands of the 5 kb Pou5f1 locus, shown as a coverage plot. Reads were strand-specific and mapped mostly to exons. Non-mRNA background was minimal as judged by hits to introns on the forward strand (top lane) or to the reverse strand (bottom lane). (C) Comparison with standard RNA-Seq (Cloonan et al. Nat. Methods 5:613-619 (2008)) for the 6 kb Nanog locus. Since cDNA synthesis was primed from the poly(A) tail, reads were typically clustered at the 3' end (top lane). The extended 3' UTR of Nanog (light blue) was clearly detected by both methods. SQRL reads extended to 5' exons, but showed more intronic background. (D) The mitochondrial genome. As expected, transcription was nearly exclusively detected on the H strand (top), with only a few truncated transcripts (arrow) originating on the L strand. Protein-coding genes are indicated on the bottom. (E) Gene expression on chromosome 19. Coverage on the forward (top lane) and reverse (middle) strands was highly correlated with local gene density (bottom). Genes are shown as horizontal stacked bars. For clarity, the vertical scales in (B-E) are truncated at half maximal values.

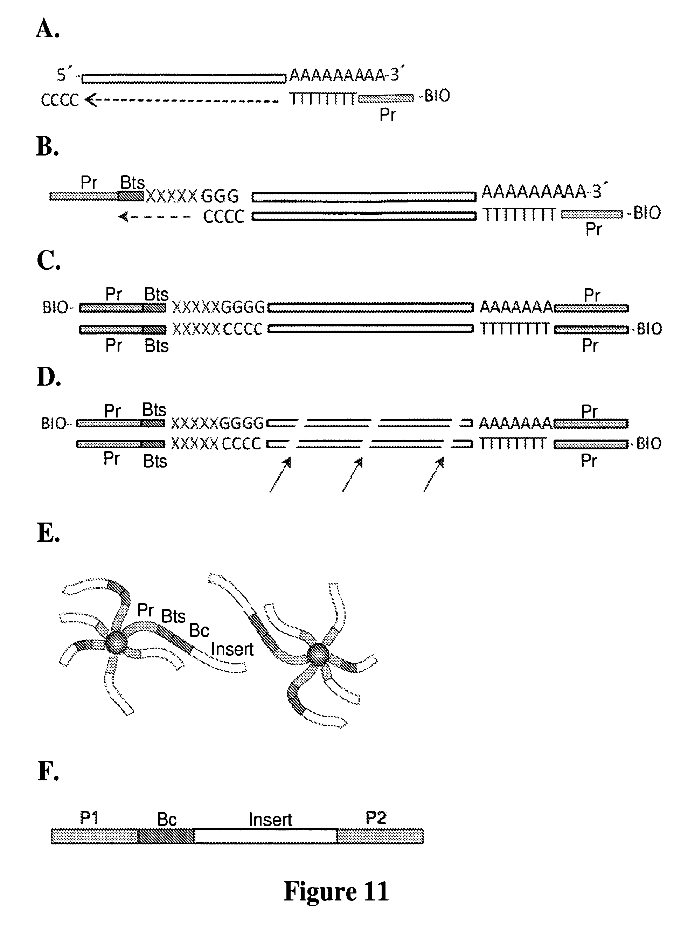

**Figure 11**, panels A-F, show graphical representation of steps optionally used in the STRT method. Panel A shows a reverse transcription step. A tailed oligo-dT primer directs synthesis of a cDNA strand. When the end of the template RNA is reached, reverse transcriptase adds 3-4 Cs at 3' of cDNA strand (due to its terminal transferase activity). Panel B shows a template switching step. Barcoded helper oligo anneals transiently, and cDNA synthesis proceeds using the oligo as template. As a consequence, the 3' end of the cDNA will carry barcode (XXXX), BtsCI recognition (Bts) and primer annealing (Pr) sequences. Panel C shows a single primer PCR step. The ends of the cDNA have identical sequences and are amplified using a single PCR primer, which helps suppress short amplicons. Panel D shows a fragmentation step. The amplified library is fragmented to 200 - 300 bp using controlled DNase digestion. Panel E shows an immobilization and end-repair step. Both 5' (with barcode (Br) and BtsCI site (Bts)) and 3' fragments are bound to beads, while internal fragments are washed away. Panel F shows fragment release and adapter ligation. 5' fragments are released by BtsCI digestion, leaving just the barcode (Bc) and insert (white area). 3' fragments remain stuck on the beads. Genome Analyzer paired-end compatible adapters (P1 and P2) are ligated. The library is sequenced from the P1 primer (and could optionally be sequenced also from P2). P1 reads begin with a 5 bp barcode, followed by 3-4 Gs, followed by the cDNA insert. P2 reads would yield only cDNA sequence.

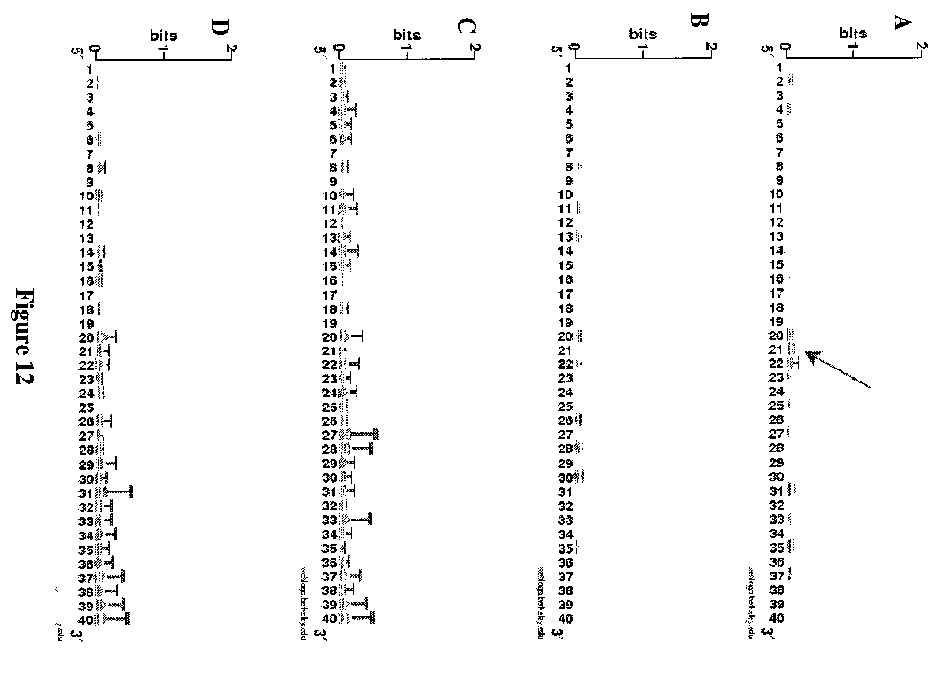

**Figure 12****,** panels A-D, show a graphical representation of an absence of motifs surrounding the template-switching site. All reads were examined in sample L006 for the presence of any motif around the template-switching site (that is, around the 5' end of each read). Sequence logos are shown for the 20 bases of genomic sequence upstream and downstream of the first nucleotide (arrow) of each mapped read. As exemplified in A and B, in typical cases (92 of 96), no strong motif was detected, indicating an absence of significant mispriming events, which would have generated an upstream motif complementary to the primer. In four cases (C and D), there was a general preference for T-rich sequences particularly in the first 20 bases of the read. This occurred in wells with very small numbers of reads, indicating a failed reaction. However, in a single case the T-rich motif was observed despite a large number of reads.

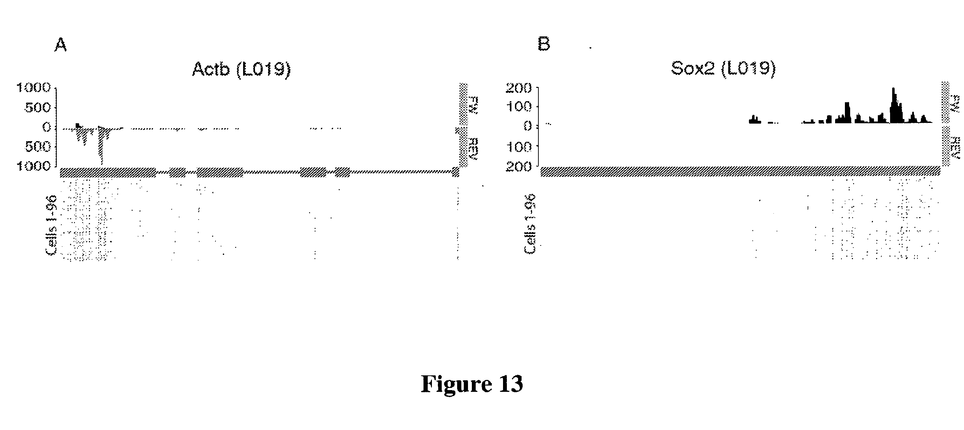

**Figure 13****,** panels A and B, show a graphical representation of hotspots for template switching. (A) The Actb locus is expressed from the lower (reverse) strand, right to left in the figure. The top two tracks show aggregate hits on the forward and reverse strands, respectively, demonstrating strand specificity and lack of background in introns. The middle (blue) track shows the exon/intron structure of the gene. The lower track shows individual hits from single cells. Each pixel row represents the hits from a single cell as black dots. There are 96 pixel rows altogether. (B) The same analysis was done for Sox2, a single-exon gene transcribed on the forward (upper) strand, showing the usual 3' bias. In both (A) and (B), hotspots were clearly visible, and were shared among cells, suggesting they represent structural sites on mRNA that favor termination of cDNA synthesis, RNA hydrolysis and/or template switching.

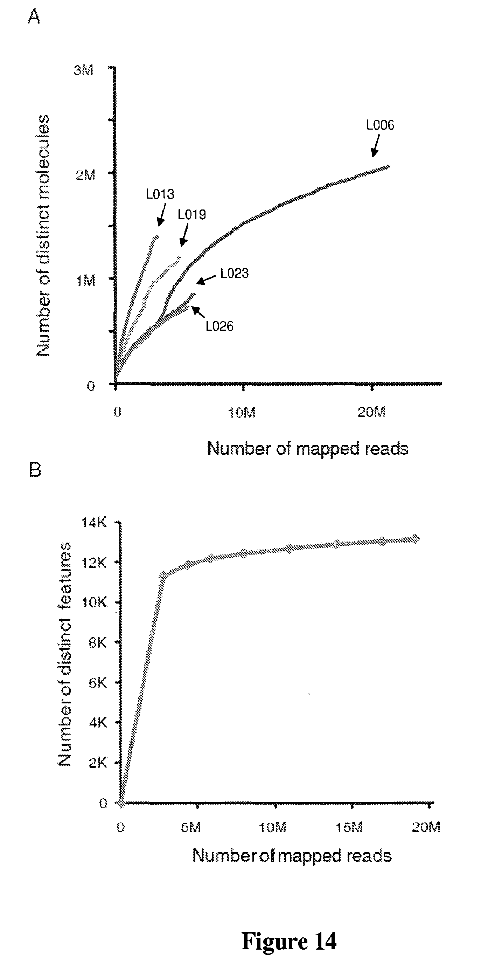

**Figure 14****,** panels A and B, show a graphical representation of the new discovery rate. (A) shows the rate of discovery of distinct mapping reads as a function of the total number of reads. None of the samples were sequenced to saturation, and most libraries would likely contain at least 3 million distinct molecules, indicating that on average at least 30,000 distinct molecules per cell were successfully converted to amplifiable cDNA. The curves are wiggly due to inhomogeneities in the data, presumably caused by imperfections in the cluster PCR process that can generate local duplicates and thus less than random sampling. In (B) the rate of discovery of distinct annotated features is shown as a function of the number of mapped reads (for sample L006). Saturation was reached quickly, showing that most features present in the sample could be discovered at modest sampling depth.

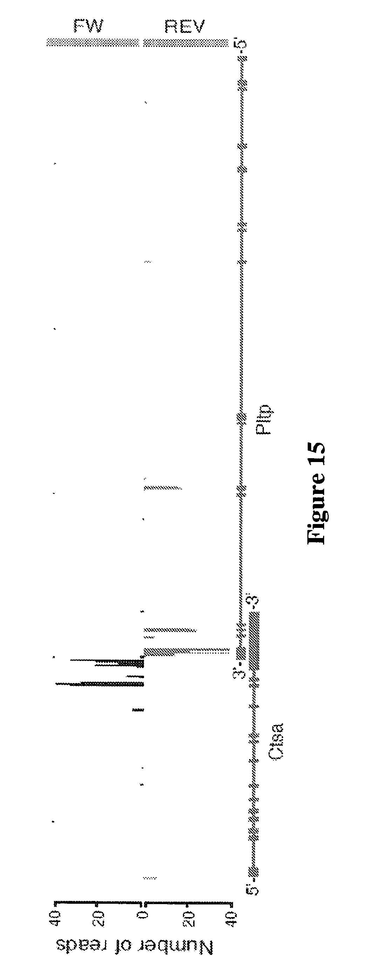

**Figure 15** shows a graphical representation for distinguishing expression of overlapping genes. Because of the strand-specificity of the template-switching mechanism, strandedness could be maintained throughout the protocol. This was especially important for genes with overlapping exons. Represented in the figure is an example of such a pair of genes, Cathepsin A (Ctsa) and Phospholipid transfer protein (Pltp), whose last exons overlap. Without strand information, the reads in the last four exons of Pltp could not be distinguished from the reads originating in the last exon of Ctsa. There are about 3,000 genes with similar overlapping 3' exons.

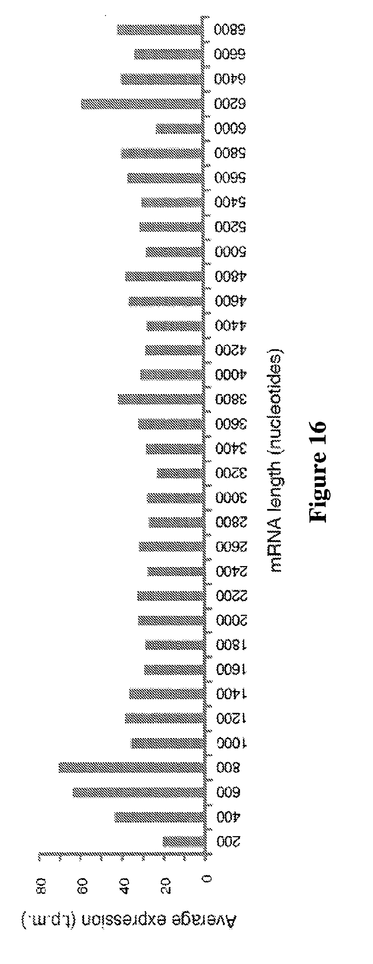

**Figure 16** shows a graphical representation of length bias for transcripts. In order to detect any bias against short or long transcripts, the average expression level was calculated as a function of mRNA length (in 200 bp bins) for sample L019. Each bar shows the expression level of genes with transcripts shorter than the indicated length (thus the first bar contains transcripts 0 - 200 bp long). Over a wide range of mRNA length, there was no apparent difference in measured expression levels. The shortest transcripts (<200 bp) were presumably suppressed by the gel purification step where inserts >100 bp were selected. The apparent overexpression of genes in the 400 - 800 bp range can possibly be explained by a more efficient template switching in this range, where cDNA synthesis would often reach the 5' cap of mRNA. Alternatively, it can simply be due to the presence of a few very highly expressed genes in this range, including Dppa5 and Rps14.

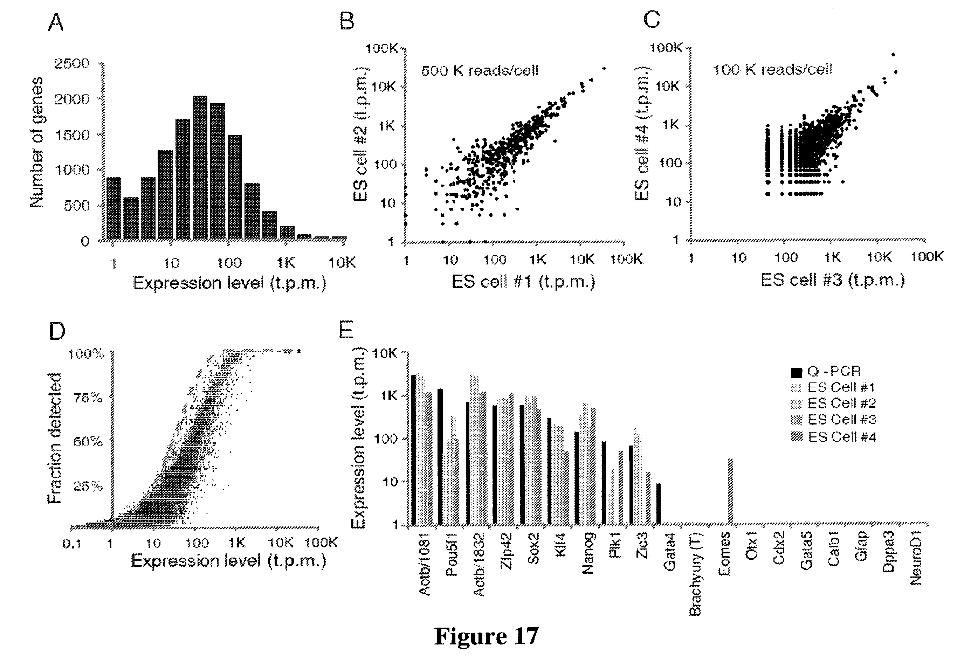

**Figure 17**, panels A-E, show a graphical representation of the quantitative accuracy of the STRT method. (A) Distribution of gene expression levels, in transcripts per million (t.p.m.) showed predominantly low expression, in the 10 - 100 t.p.m. range. (B) Comparison of two cells sequence to a depth of approximately 500 000 reads/cell. In this case, genes below 100 t.p.m. could be accurately quantified. (C) Comparison of two cells sequenced to approximately 100 000 reads/cell. In this case, sensitivity dropped to about 1000 t.p.m. (D) Probability of detection as a function of expression level. Each dot shows a gene, with a given average expression level (across all cells) and fraction of cells having non-zero expression of this gene. The distribution approach the theoretical limit of random sampling given the actual depth of sequencing used here (dashed line). (E) Comparison with quantitative real-time PCR for the four ES cells shown in (B) and (C), using select markers of pluripotency and differentiation. In general, the quantitative accuracy was good, with a single putative false positive (Eomes in ES Cell #4). However this was a rare event, and was not observed for this gene in any of the other 160 ES cells examined. Q-PCR levels were converted to t.p.m. by normalization of the Actb/1081 primer pair with ES cells #1 and #2. Actin was then independently measured using a different primer pair (Actb/1832) to confirm the accuracy of the normalization.

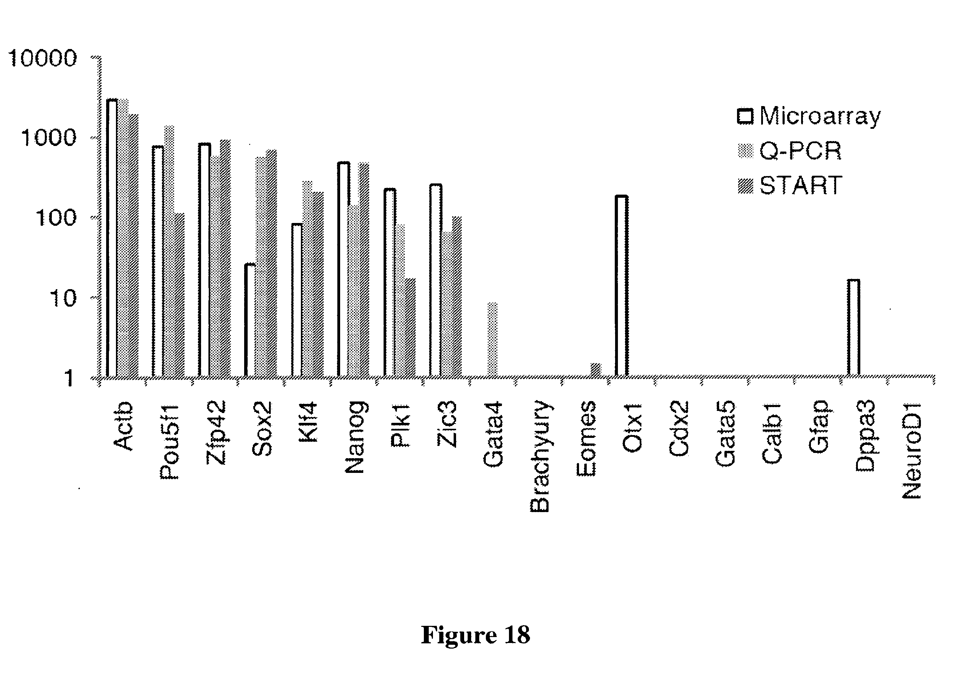

**Figure 18** shows a graphical comparison between STRT, Q-PCR and microarray analyses. Genes expected to be expressed (Actb, Pou5f1, Zfp42, Sox2, Klf4, Nanog, Plk1, Zic3) or not expressed (Gata4, Brachyury, Eomes, Otx1, Cdx2, Gata5, Calb1, Gfap, Dppa3 and NeuroD1) in undifferentiated ES cells were analyzed by STRT, quantitative real-time PCR (Q-PCR) and Illumina microarray. There was good correlation between STRT and Q-PCR, and to a lesser extent with microarray data. In particular, Sox2 was undercalled on the microarray, while Otx1 and Dppa3 were apparent false positives. The microarray data is the mean of two hybridization reactions, Q-PCR was performed in duplicate and repeated once for confirmation, and the STRT data is the mean of 160 single ES cells.

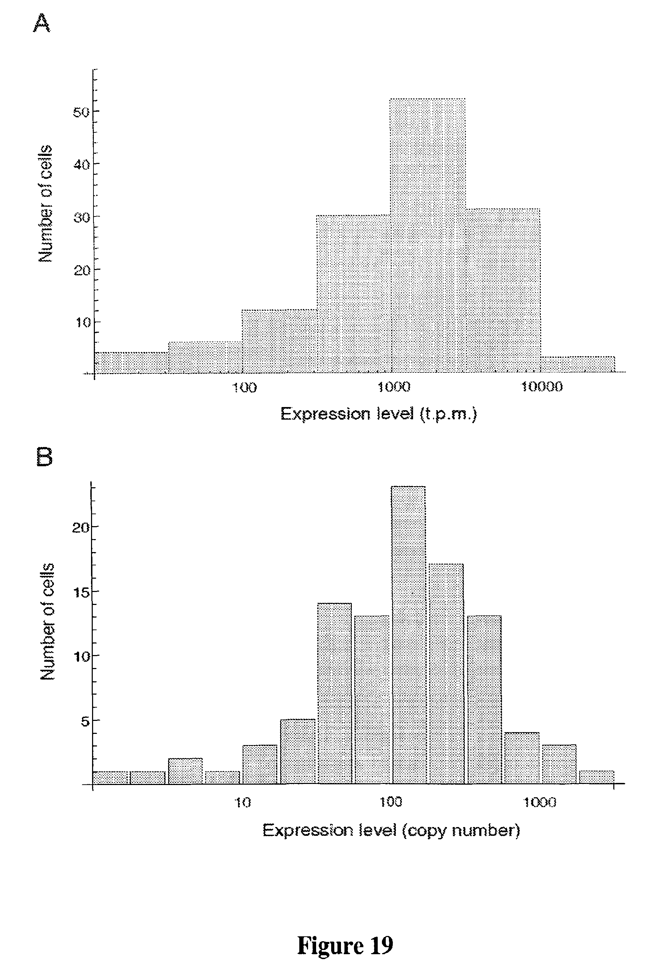

**Figure 19****,** panels A and B, show a graphical representation of gene expression distribution across cells. (A) shows the distribution of Actb expression levels across all cells using STRT. (B) shows Actb expression in pancreatic islet cells from Bengtsson et al. Genome Res 15(10):1388-92 (2005) for comparison.

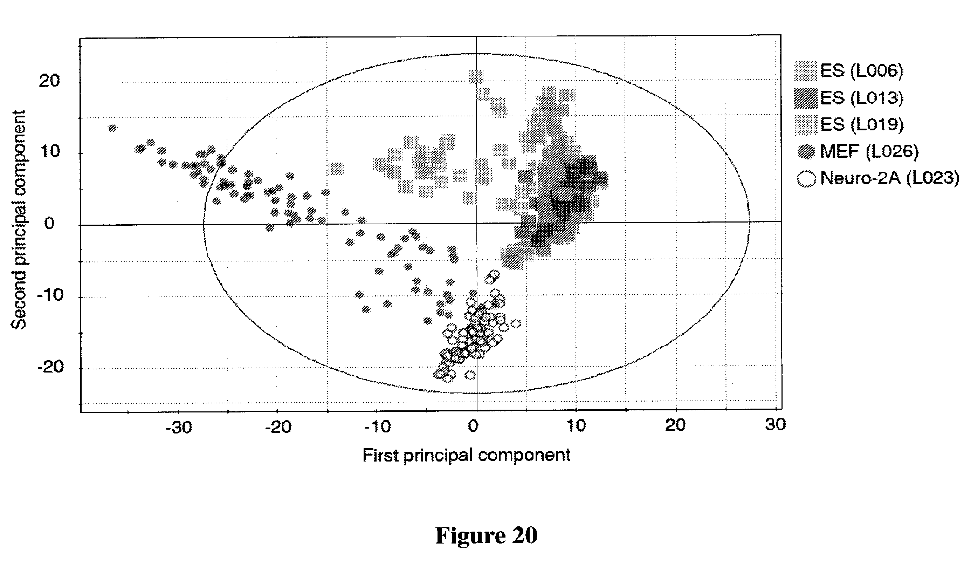

**Figure 20** shows a principal component analysis. In order to discover and cluster cell types based on expression data, the five independently prepared 96-cell samples were subjected to principal component analysis. The three types of cells (ES, Neuro-2A and MEF) clearly clustered separately, although MEFs did not form a very distinct cluster. Moreover, ES cells prepared independently clustered together, showing that the PCA did not simply pick up sample preparation differences. This demonstrates that single-cell expression data can be used to accurately classify cell types.

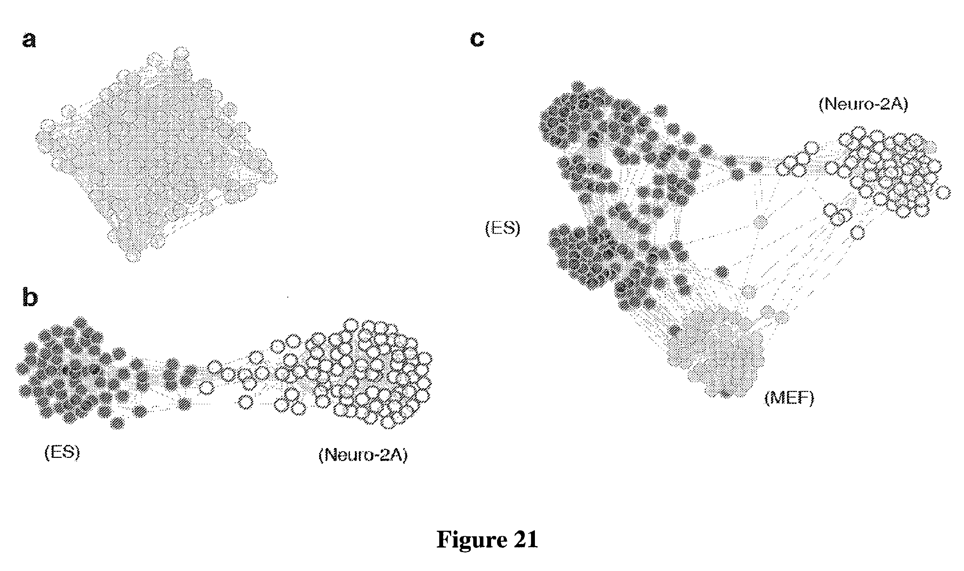

**Figure 21**, panels A-C, show a graph-based visualization ("cell map") of the expression pattern. (A) Cells, represented by graph nodes (circles) were laid out randomly, and edges were drawn from each cell to the five other cells it was most highly correlated with. (B) Force-directed layout was used to lay out the graph on a plane. In this layout, cells repelled each other uniformly, but were held together by edges acting as elastic springs. The resulting visual map was consistent with known cell identities (ES cells vs Neuro-2A cells), with some less deeply sequenced cells showing poor separation. (C) Adding more ES cells as well as fibroblasts (MEFs) expanded the map and demonstrated that independently prepared ES cells grouped together accurately.

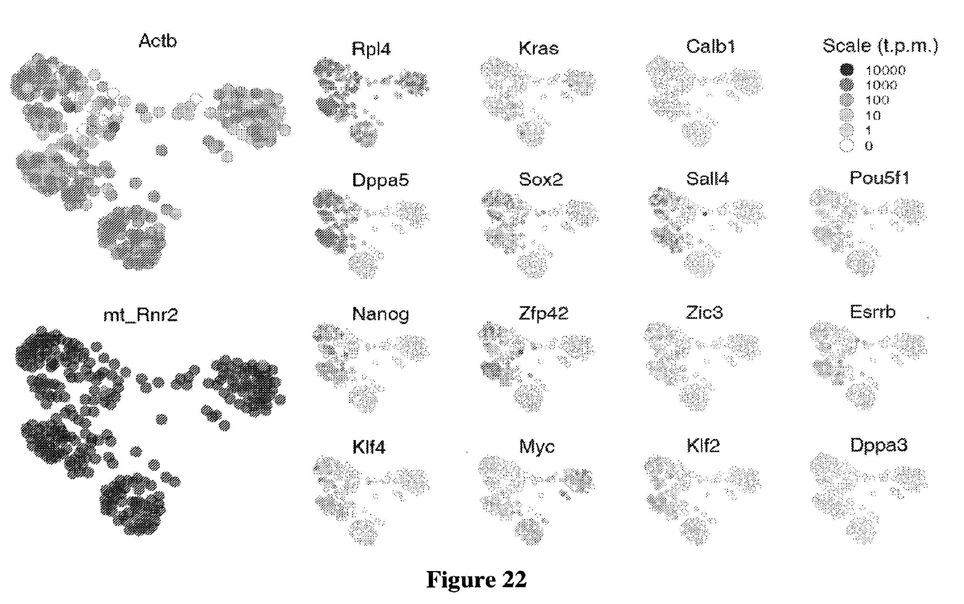

**Figure 22** shows the visualizing of gene expression on a cell map of Figure 21. Each map retains its layout from **Figure 21C****,** but cells are shaded according to expression of the indicated gene. A logarithmic scale was used (upper right). Mitochondrial ribosomal RNA 2 (mt_Rnr2) was the highest-expressed gene of all. Housekeeping genes such as Actin (Actb) and ribosomal protein L4 (Rpl4) were detected in all cell types, but not in every single cell. The power of shotgun single cell expression profiling was revealed for low-expressed genes like K-ras (Kras), which was detected only in approximately half of all cells, but still clearly expressed in all cell types. Calbindin (Calb1) was absent, as expected and confirmed by Q-PCR. A set of well-known ES cell markers (Dppa5, Sox2, Sall4. Pou5f1, Nanog, Zfp42, Zic3 and Esrrb) were clearly expressed specifically in the ES cell cluster, whereas Klf4, Myc and Klf2 were more widely distributed. Dppa3 was not detected, as confirmed by Q-PCR **(****Figure 18****)**

### DETAILED DESCRIPTION OF THE INVENTION

The present invention provides methods and compositions for the analysis of gene expression in single cells or in a plurality of single cells. In particular, the invention provides methods for preparing a cDNA library from a plurality of single cells. The methods are based on determining gene expression levels from a population of individual cells, which can be used to identify natural variations in gene expression on a cell by cell level. The methods can also be used to identify and characterize the cellular composition of a population of cells in the absence of suitable cell-surface markers. The methods described herein also provide the advantage of generating a DNA library representative of RNA content in a cell population by using single cells, whereas cDNA libraries prepared by classical methods typically require total RNA isolated from a large population (see Example I). Thus, a cDNA library produced using the methods of the invention provide at least equivalent representation of RNA content in a population of cells by utilizing a smaller subpopulation of individual cells along with additional advantages as described herein.

Embodiments also provide sampling of a large number of single cells. Using similarity of expression patterns, a map of cells can be built showing how the cells relate. This map can be used to distinguish cell types *in silico,* by detecting clusters of closely related cells (see Example II). By sampling not just a few, but large numbers of single cells, similarity of expression patterns can be used to build a map of cells and how they are related. This method permits access to undiluted expression data from every distinct type of cell present in a population, without the need for prior purification of those cell types. In addition, where known markers are available, these can be used *in silico* to delineate cells of interest. The validity of this approach is shown in Example II, which analyzes a collection of cells sampled from three distinct cell types (mouse embryonic stem cells, embryonic fibroblasts and neuroblastoma cells) of distinct embryonic origins (pluripotent stem cells vs. mesodermal and ectodermal germ layers) and disease state (normal vs. transformed).

Embodiments of the invention provide a method of preparing a cDNA library from a plurality of single cells by releasing mRNA from each single cell to provide a plurality of individual samples, wherein the mRNA in each individual mRNA sample is from a single cell, synthesizing a first strand of cDNA from the mRNA in each individual mRNA sample and incorporating a tag into the cDNA to provide a plurality of tagged cDNA samples, wherein the cDNA in each tagged cDNA sample is complementary to mRNA from a single cell pooling the tagged cDNA samples and amplifying the pooled cDNA samples to generate a cDNA library comprising double-stranded cDNA. By utilizing the above method, it is feasible to prepare samples for sequencing from several hundred single cells in a short time and with a minimal amount of work. Traditional methods for preparing a fragment library from RNA for sequencing include gel excision steps that are laborious. In the absence of special equipment, it is not convenient to prepare more than a handful of samples in parallel. In some aspects of the methods described herein, a set of 96 cells is prepared as a single sample (after cDNA synthesis), which makes it feasible to prepare several hundred cells for sequencing. Additionally, technical variation is minimized because each set of 96 cells is prepared together (in a single tube).

In some aspects of the invention, each cDNA sample obtained from a single cell is tagged, which allows gene expression to be analyzed at the level of a single cell. This allows dynamic processes, such as the cell cycle, to be studied and distinct cell types in a complex tissue (e.g. the brain) to be analyzed. In some aspects, the cDNA samples can be pooled prior to analysis. Pooling the samples simplifies handling of the samples from each single cell and reduces the time required to analyze gene expression in the single cells, which allows for high throughput analysis of gene expression. Pooling of the cDNA samples prior to amplification also provides the advantage that technical variation between samples is virtually eliminated. In addition, as the cDNA samples are pooled before amplification, less amplification is required to generate sufficient amounts of cDNA for subsequent analysis compared to amplifying and treating cDNA samples from each single cell separately. This reduces amplification bias, and also means that any bias will be similar across all the cells used to provide pooled cDNA samples. RNA purification, storage and handling are also not required, which helps to eliminate problems caused by the unstable nature of RNA.

As the cDNA libraries produced by the method of the invention are suitable for the analysis the gene expression profiles of single cells by direct sequencing, it is possible to use these libraries to study the expression of genes which were not previously known, and also to analyze alternative splicing, promoters and polyadenylation signals. Preparing the cDNA libraries as described herein, provides for a sensitive method for detecting a single or low copy RNA transcript. The sensitivity of the method is shown in **Figure 17D** and described in Example II. For example, genes expressed at 100 transcripts per million (t.p.m.) are detected about 50% of the time. However, as shown in **Figure 14A****,** the samples were not saturated, so there is additional sensitivity that is achievable with deeper sequencing of the samples. Accordingly, the method for preparing the cDNA libraries as described herein detect a single or low copy RNA transcript at least 30% of the time, or alternatively at least 40% of the time, or at least 50% of the time, or alternatively at least 60% of the time, or alternatively at least 70 % of the time, or alternatively at least 80% of the time or alternatively at least 90% of the time, or alternatively at least 95% of the time.

Embodiments also provide a method for identifying a single cell type out of a sample and/or determining the transcriptome of a single cell by preparing a cDNA library as described herein, determining the expression levels of individual cells in a population, and mapping of the individual cells based on similarity of expression patterns. Mapping of individual cells can be done *in silico* by one of skill in the art and in particular utilizing the methods described herein, such as shown in Example II. The number of cells needed to determine the frequency of a given cell type in the plurality of cells will follow a binomial distribution. For example, a predetermined number of individual cells can be sampled so that at least ten of the desired type are expected to be detected. Accordingly, if the frequency of the cell type in the sample is 10%, a cDNA library from approximately 100 cells will need to be prepared and analyzed as described herein.

The term "cDNA library" refers to a collection of cloned complementary DNA (cDNA) fragments, which together constitute some portion of the transcriptome of a single cell or a plurality of single cells. cDNA is produced from fully transcribed mRNA found in a cell and therefore contains only the expressed genes of a single cell or when pooled together the expressed genes from a plurality of single cells.

As used herein, a "plurality" refers to a population of cells and can include any number of cells desired to be analyzed. In some aspects, a plurality of cells includes at least 10 cells, or alternatively at least 25 cells, or alternatively at least 50 cells, or alternatively at least 100 cells, or alternatively at least 200 cells, or alternatively at least 500 cells, or alternatively at least 1000 cells, or alternatively 5,000 cells or alternatively 10,000 cells. In another aspect, a plurality of cells includes from 10 to 100 cells, or alternatively from 50 to 200 cells, alternatively from 100 to 500 cells, or alternatively from 100 to 1000, or alternatively from 1,000 to 5,000 cells.

The expression "amplification" or "amplifying" refers to a process by which extra or multiple copies of a particular polynucleotide are formed. Amplification includes methods such as PCR, ligation amplification (or ligase chain reaction, LCR) and amplification methods. These methods are known and widely practiced in the art. See, e.g., U.S. Patent Nos. 4,683,195 and 4,683,202 and Innis et al., "PCR protocols: a guide to method and applications" Academic Press, Incorporated (1990) (for PCR); and Wu et al. (1989) Genomics 4:560-569 (for LCR). In general, the PCR procedure describes a method of gene amplification which is comprised of (i) sequence-specific hybridization of primers to specific genes within a DNA sample (or library), (ii) subsequent amplification involving multiple rounds of annealing, elongation, and denaturation using a DNA polymerase, and (iii) screening the PCR products for a band of the correct size. The primers used are oligonucleotides of sufficient length and appropriate sequence to provide initiation of polymerization, i.e. each primer is specifically designed to be complementary to each strand of the genomic locus to be amplified.

Reagents and hardware for conducting amplification reaction are commercially available. Primers useful to amplify sequences from a particular gene region are preferably complementary to, and hybridize specifically to sequences in the target region or in its flanking regions and can be prepared using the polynucleotide sequences provided herein. Nucleic acid sequences generated by amplification can be sequenced directly.

When hybridization occurs in an antiparallel configuration between two single-stranded polynucleotides, the reaction is called "annealing" and those polynucleotides are described as "complementary". A double-stranded polynucleotide can be complementary or homologous to another polynucleotide, if hybridization can occur between one of the strands of the first polynucleotide and the second. Complementarity or homology (the degree that one polynucleotide is complementary with another) is quantifiable in terms of the proportion of bases in opposing strands that are expected to form hydrogen bonding with each other, according to generally accepted base-pairing rules.

As used herein, a "single cell" refers to one cell. Single cells useful in the methods described herein can be obtained from a tissue of interest, or from a biopsy, blood sample, or cell culture. Additionally, cells from specific organs, tissues, tumors, neoplasms, or the like can be obtained and used in the methods described herein. Furthermore, in general, cells from any population can be used in the methods, such as a population of prokaryotic or eukaryotic single celled organisms including bacteria or yeast. In some aspects, the method of preparing the cDNA library can include the step of obtaining single cells. A single cell suspension can be obtained using standard methods known in the art including, for example, enzymatically using trypsin or papain to digest proteins connecting cells in tissue samples or releasing adherent cells in culture, or mechanically separating cells in a sample. Single cells can be placed in any suitable reaction vessel in which single cells can be treated individually. For example a 96-well plate, such that each single cell is placed in a single well.

Methods for manipulating single cells are known in the art and include fluorescence activated cell sorting (FACS), micromanipulation and the use of semi-automated cell pickers (e.g. the Quixell™ cell transfer system from Stoelting Co.). Individual cells can, for example, be individually selected based on features detectable by microscopic observation, such as location, morphology, or reporter gene expression.

In some aspects, mRNA can be released from the cells by lysing the cells. Lysis can be achieved by, for example, heating the cells, or by the use of detergents or other chemical methods, or by a combination of these. However, any suitable lysis method known in the art can be used. A mild lysis procedure can advantageously be used to prevent the release of nuclear chromatin, thereby avoiding genomic contamination of the cDNA library, and to minimise degradation of mRNA. For example, heating the cells at 72°C for 2 minutes in the presence of Tween-20 is sufficient to lyse the cells while resulting in no detectable genomic contamination from nuclear chromatin. Alternatively, cells can be heated to 65°C for 10 minutes in water (Esumi et al., Neurosci Res 60(4):439-51 (2008)); or 70°C for 90 seconds in PCR buffer II (Applied Biosystems) supplemented with 0.5% NP-40 (Kurimoto et al., Nucleic Acids Res 34(5):e42 (2006)); or lysis can be achieved with a protease such as Proteinase K or by the use of chaotropic salts such as guanidine isothiocyanate (U.S. Publication No. 2007/028)313).

Synthesis of cDNA from mRNA in the methods described herein can be performed directly on cell lysates, such that a reaction mix for reverse transcription is added directly to cell lysates. Alternatively, mRNA can be purified after its release from cells. This can help to reduce mitochondrial and ribosomal contamination. mRNA purification can be achieved by any method known in the art, for example, by binding the mRNA to a solid phase. Commonly used purification methods include paramagnetic beads (e.g. Dynabeads). Alternatively, specific contaminants, such as ribosomal RNA can be selectively removed using affinity purification.

cDNA is typically synthesized from mRNA by reverse transcription. Methods for synthesizing cDNA from small amounts of mRNA, including from single cells, have previously been described (Kurimoto et al., Nucleic Acids Res 34(5):e42 (2006); Kurimoto et al., Nat Protoc 2(3):739-52 (2007); and Esumi et al., Neurosci Res 60(4):439-51 (2008)). In order to generate an amplifiable cDNA, these methods introduce a primer annealing sequence at both ends of each cDNA molecule in such a way that the cDNA library can be amplified using a single primer. The Kurimoto method uses a polymerase to add a 3' poly-A tail to the cDNA strand, which can then be amplified using a universal oligo-T primer. In contrast, the Esumi method uses a template switching method to introduce an arbitrary sequence at the 3' end of the cDNA, which is designed to be reverse complementary to the 3' tail of the cDNA synthesis primer. Again, the cDNA library can be amplified by a single PCR primer. Single-primer PCR exploits the PCR suppression effect to reduce the amplification of short contaminating amplicons and primer-dimers (Dai et al., J Biotechnol 128(3):435-43 (2007)). As the two ends of each amplicon are complementary, short amplicons will form stable hairpins, which are poor templates for PCR. This reduces the amount of truncated cDNA and improves the yield of longer cDNA molecules.

In some aspects of the invention, the synthesis of the first strand of the cDNA can be directed by a cDNA synthesis primer (CDS) that includes an RNA complementary sequence (RCS). In some aspects of the invention, the RCS is at least partially complementary to one or more mRNA in an individual mRNA sample. This allows the primer, which is typically an oligonucleotide, to hybridize to at least some mRNA in an individual mRNA sample to direct cDNA synthesis using the mRNA as a template. The RCS can comprise oligo (dT), or be gene family-specific, such as a sequence of nucleic acids present in all or a majority related genes, or can be composed of a random sequence, such as random hexamers. To avoid the CDS priming on itself and thus generating undesired side products, a non-self-complementary semi-random sequence can be used. For example, one letter of the genetic code can be excluded, or a more complex design can be used while restricting the CDS to be non-self-complementary.

The terms "oligonucleotide" and "polynucleotide" are used interchangeably and refer to a polymeric form of nucleotides of any length, either deoxyribonucleotides or ribonucleotides or analogs thereof. Polynucleotides can have any three-dimensional structure and can perform any function, known or unknown. The following are non-limiting examples of polynucleotides: a gene or gene fragment (for example, a probe, primer, EST or SAGE tag), exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes and primers. A polynucleotide can comprise modified nucleotides, such as methylated nucleotides and nucleotide analogs. The term also refers to both double- and single-stranded molecules. Unless otherwise specified or required, any embodiment that comprises a polynucleotide encompasses both the double-stranded form and each of two complementary single-stranded forms known or predicted to make up the double-stranded form.

A polynucleotide is composed of a specific sequence of four nucleotide bases: adenine (A); cytosine (C); guanine (G); thymine (T); and uracil (U) for thymine when the polynucleotide is RNA. Thus, the term polynucleotide sequence is the alphabetical representation of a polynucleotide molecule. This alphabetical representation can be input into databases in a computer having a central processing unit and used for bioinformatics applications such as functional genomics and homology searching.

A "primer" is a short polynucleotide, generally with a free 3' -OH group that binds to a target or template potentially present in a sample of interest by hybridizing with the target, and thereafter promoting polymerization of a polynucleotide complementary to the target. Primers of the instant invention are comprised of nucleotides ranging from 17 to 30 nucleotides. In one aspect, the primer is at least 17 nucleotides, or alternatively, at least 18 nucleotides, or alternatively, at least 19 nucleotides, or alternatively, at least 20 nucleotides, or alternatively, at least 21 nucleotides, or alternatively, at least 22 nucleotides, or alternatively, at least 23 nucleotides, or alternatively, at least 24 nucleotides, or alternatively, at least 25 nucleotides, or alternatively, at least 26 nucleotides, or alternatively, at least 27 nucleotides, or alternatively, at least 28 nucleotides, or alternatively, at least 29 nucleotides, or alternatively, at least 30 nucleotides, or alternatively at least 50 nucleotides, or alternatively at least 75 nucleotides or alternatively at least 100 nucleotides.

The RCS can also be at least partially complementary to a portion of the first strand of cDNA, such that it is able to direct the synthesis of a second strand of cDNA using the first strand of the cDNA as a template. Thus, following first strand synthesis, an RNase enzyme (e.g. an enzyme having RNaseH activity) can be added after synthesis of the first strand of cDNA to degrade the RNA strand and to permit the CDS to anneal again on the first strand to direct the synthesis of a second strand of cDNA. For example, the RCS could comprise random hexamers, or a non-self complementary semi-random sequence (which minimizes self-annealing of the CDS).

A template-switching oligonucleotide (TSO) that includes a portion which is at least partially complementary to a portion of the 3' end of the first strand ofcDNA can be added to each individual mRNA sample in the methods described herein. Such a template switching method is described in (Esumi et al., Neurosci Res 60(4):439-51 (2008)) and allows full length cDNA comprising the complete 5' end of the mRNA to be synthesized. As the terminal transferase activity of reverse transcriptase typically causes 2-5 cytosines to be incorporated at the 3' end of the first strand of cDNA synthesized from mRNA, the first strand of cDNA can include a plurality of cytosines, or cytosine analogues that base pair with guanosine, at its 3' end (see US 5,962,272). In one aspect of the invention, the first strand of cDNA can include a 3' portion comprising at least 2, at least 3, at least 4, at least 5 or 2, 3, 4, or 5 cytosines or cytosine analogues that base pair with guanosine. A non-limiting example of a cytosine analogue that base pairs with guanosine is 5-aminoallyl-2'-deoxycytidine.

In one aspect of the invention, the TSO can include a 3' portion comprising a plurality of guanosines or guanosine analogues that base pair with cytosine. Non-limiting examples of guanosines or guanosine analogues useful in the methods described herein include, but are not limited to, deoxyriboguanosine, riboguanosine, locked nucleic acid-guanosine, and peptide nucleic acid-guanosine. The guanosines can be ribonucleosides or locked nucleic acid monomers.

A locked nucleic acid (LNA) is a modified RNA nucleotide. The ribose moiety of an LNA nucleotide is modified with an extra bridge connecting the 2' oxygen and 4' carbon. The bridge "locks" the ribose in the 3'-endo (North) conformation. Some of the advantages of using LNAs in the methods of the invention include increasing the thermal stability of duplexes, increased target specificity and resistance from exo- and endonucleases.

A peptide nucleic acid (PNA) is an artificially synthesized polymer similar to DNA or RNA, wherein the backbone is composed of repeating N-(2-aminoethyl)-glycine units linked by peptide bonds. The backbone of a PNA is substantially non-ionic under neutral conditions, in contrast to the highly charged phosphodiester backbone of naturally occurring nucleic acids. This provides two non-limiting advantages. First, the PNA backbone exhibits improved hybridization kinetics. Secondly, PNAs have larger changes in the melting temperature (Tm) for mismatched versus perfectly matched basepairs. DNA and RNA typically exhibit a 2-4° C drop in Tm for an internal mismatch. With the non-ionic PNA backbone, the drop is closer to 7-9° C. This can provide for better sequence discrimination. Similarly, due to their non-ionic nature, hybridization of the bases attached to these backbones is relatively insensitive to salt concentration.

A nucleic acid useful in the invention can contain a non-natural sugar moiety in the backbone. Exemplary sugar modifications include but are not limited to 2' modifications such as addition of halogen, alkyl, substituted alkyl, SH, SCH₃, OCN, Cl, Br, CN, CF₃, OCF₃, SO₂CH₃, OSO₂, SO₃, CH₃, ONO₂, NO₂, N₃, NH₂, substituted silyl, and the like. Similar modifications can also be made at other positions on the sugar, particularly the 3' position of the sugar on the 3' terminal nucleotide or in 2'-5' linked oligonucleotides and the 5' position of 5' terminal nucleotide. Nucleic acids, nucleoside analogs or nucleotide analogs having sugar modifications can be further modified to include a reversible blocking group, peptide linked label or both. In those embodiments where the above-described 2' modifications are present, the base can have a peptide linked label.

A nucleic acid used in the invention can also include native or non-native bases. In this regard a native deoxyribonucleic acid can have one or more bases selected from the group consisting of adenine, thymine, cytosine or guanine and a ribonucleic acid can have one or more bases selected from the group consisting of uracil, adenine, cytosine or guanine. Exemplary non-native bases that can be included in a nucleic acid, whether having a native backbone or analog structure, include, without limitation, inosine, xathanine, hypoxathanine, isocytosine, isoguanine, 5-methylcytosine, 5-hydroxymethyl cytosine, 2-aminoadenine, 6-methyl adenine, 6-methyl guanine, 2-propyl guanine, 2-propyl adenine, 2-thioLiracil, 2-thiothymine, 2-thiocytosine, 15-halouracil, 15-halocytosine, 5-propynyl uracil, 5-propynyl cytosine, 6-azo uracil, 6-azo cytosine, 6-azo thymine, 5-uracil, 4-thiouracil, 8-halo adenine or guanine, 8-amino adenine or guanine, 8-thiol adenine or guanine, 8-thioalkyl adenine or guanine, 8-hydroxyl adenine or guanine, 5-halo substituted uracil or cytosine, 7-methylguanine, 7-methyladenine, 8-azaguanine, 8-azaadenine, 7-deazaguanine, 7-deazaadenine, 3-deazaguanine, 3-deazaadenine or the like. A particular embodiment can utilize isocytosine and isoguanine in a nucleic acid in order to reduce non-specific hybridization, as generally described in U.S. Pat. No. 5,681,702.

A non-native base used in a nucleic acid can have universal base pairing activity, wherein it is capable of base pairing with any other naturally occurring base. Exemplary bases having universal base pairing activity include 3-nitropyrrole and 5-nitroindole. Other bases that can be used include those that have base pairing activity with a subset of the naturally occurring bases such as inosine, which basepairs with cytosine, adenine or uracil.

In one aspect of the invention, the TSO can include a 3' portion including at least 2, at least 3, at least 4, at least 5, or 2, 3, 4, or 5, or 2-5 guanosines, or guanosine analogues that base pair with cytosine. The presence of a plurality of guanosines (or guanosine analogues that base pair with cytosine) allows the TSO to anneal transiently to the exposed cytosines at the 3' end of the first strand of cDNA. This causes the reverse transcriptase to switch template and continue to synthesis a strand complementary to the TSO. In one aspect of the invention, the 3' end of the TSO can be blocked, for example by a 3' phosphate group, to prevent the TSO from functioning as a primer during cDNA synthesis.

In one aspect, the mRNA is released from the cells by cell lysis. If the lysis is achieved partially by heating, then the CDS and/or the TSO can be added to each individual mRNA sample during cell lysis, as this will aid hybridization of the oligonucleotides. In some aspects, reverse transcriptase can be added after cell lysis to avoid denaturation of the enzyme.

In some aspects of the invention, a tag can be incorporated into the cDNA during its synthesis. For example, the CDS and/or the TSO can include a tag, such as a particular nucleotide sequence, which can be at least 4, at least 5, at least 6, at least 7, at least 8, at least 9, at least 10, at least 15 or at least 20 nucleotides in length. For example, the tag can be a nucleotide sequence of 4-20 nucleotides in length, e.g. 4, 5, 6, 7, 8, 9, 10, 15 or 20 nucleotides in length. As the tag is present in the CDS and/or the TSO it will be incorporated into the cDNA during its synthesis and can therefore act as a "barcode" to identify the cDNA. Both the CDS and the TSO can include a tag. The CDS and the TSO can each include a different tag, such that the tagged cDNA sample comprises a combination of tags. Each cDNA sample generated by the above method can have a distinct tag, or a distinct combination of tags, such that once the tagged cDNA samples have been pooled, the tag can be used to identify which single cell from each cDNA sample originated. Thus, each cDNA sample can be linked to a single cell, even after the tagged cDNA samples have been pooled in the methods described herein.

Before the tagged cDNA samples are pooled, synthesis of cDNA can be stopped, for example by removing or inactivating the reverse transcriptase. This prevents cDNA synthesis by reverse transcription from continuing in the pooled samples. The tagged cDNA samples can optionally be purified before amplification, ether before or after they are pooled.

The pooled cDNA samples can be amplified by polymerase chain reaction (PCR) including emulsion PCR and single primer PCR in the methods described herein. For example, the cDNA samples can be amplified by single primer PCR. The CDS can comprise a 5' amplification primer sequence (APS), which subsequently allows the first strand ofcDNA to be amplified by PCR using a primer that is complementary to the 5' APS. The TSO can also comprise a 5' APS, which can be at least 70% identical, at least 80% identical, at least 90% identical, at least 95% identical, or 70%, 80%, 90% or 100% identical to the 5' APS in the CDS. This means that the pooled cDNA samples can be amplified by PCR using a single primer (i.e. by single primer PCR), which exploits the PCR suppression effect to reduce the amplifcation of short contaminating amplicons and primer-dimers (Dai et al., J Biotechnol 128(3):435-43 (2007)). As the two ends of each amplicon are complementary, short amplicons will form stable hairpins, which are poor templates for PCR. This reduces the amount of truncated cDNA and improves the yield of longer cDNA molecules. The 5' APS can be designed to facilitate downstream processing of the cDNA library. For example, if the cDNA library is to be analyzed by a particular sequencing method, e.g. Applied Biosystems' SOLiD sequencing technology, or Illumina's Genome Analyzer, the 5' APS can be designed to be identical to the primers used in these sequencing methods. For example, the 5' APS can be identical to the SOLiD P1 primer, and/or a SOLiD P2 sequence inserted in the CDS, so that the P1 and P2 sequences required for SOLiD sequencing are integral to the amplified library.

Another exemplary method for amplifying pooled cDNA includes PCR. PCR is a reaction in which replicate copies are made of a target polynucleotide using a pair of primers or a set of primers consisting of an upstream and a downstream primer, and a catalyst of polymerization, such as a DNA polymerase, and typically a thermally-stable polymerase enzyme. Methods for PCR are well known in the art, and taught, for example in MacPherson et al. (1991) PCR 1 : A Practical Approach (IRL Press at Oxford University Press). All processes of producing replicate copies of a polynucleotide, such as PCR or gene cloning, are collectively referred to herein as replication. A primer can also be used as a probe in hybridization reactions, such as Southern or Northern blot analyses.

For emulsion PCR, an emulsion PCR reaction is created by vigorously shaking or stirring a "water in oil" mix to generate millions of micron-sized aqueous compartments. The DNA library is mixed in a limiting dilution either with the beads prior to emulsification or directly into the emulsion mix. The combination of compartment size and limiting dilution of beads and target molecules is used to generate compartments containing, on average, just one DNA molecule and bead (at the optimal dilution many compartments will have beads without any target) To facilitate amplification efficiency, both an upstream (low concentration, matches primer sequence on bead) and downstream PCR primers (high concentration) are included in the reaction mix. Depending on the size of the aqueous compartments generated during the emulsification step, up to 3x10⁹ individual PCR reactions per µl can be conducted simultaneously in the same tube. Essentially each little compartment in the emulsion forms a micro PCR reactor. The average size of a compartment in an emulsion ranges from submicron in diameter to over a 100 microns, depending on the emulsification conditions.

"Identity," "homology" or "similarity" are used interchangeably and refer to the sequence similarity between two nucleic acid molecules. Identity can be determined by comparing a position in each sequence which can be aligned for purposes of comparison. When a position in the compared sequence is occupied by the same base or amino acid, then the molecules are homologous at that position. A degree of identity between sequences is a function of the number of matching or identical positions shared by the sequences. An unrelated or non-homologous sequence shares less than 40% identity, or alternatively less than 25% identity, with one of the sequences.

A polynucleotide has a certain percentage (for example, 60%, 65%, 70%, 75%, 80%, 85%, 90%, 95%, 98% or 99%) of "sequence identity" to another sequence means that, when aligned, that percentage of bases are the same in comparing the two sequences. This alignment and the percent sequence identity or homology can be determined using software programs known in the art, for example those described in Ausubel et al., Current Protocols in Molecular Biology, John Wiley & Sons, New York, N.Y., (1993). Preferably, default parameters are used for alignment. One alignment program is BLAST, using default parameters. In particular, programs are BLASTN and BLASTP, using the following default parameters: Genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + SwissProtein + SPupdate + PIR. Details of these programs can be found at the National Center for Biotechnology Information.

The method of preparing a cDNA library described herein can further comprise processing the cDNA library to obtain a library suitable for sequencing. As used herein, a library is suitable for sequencing when the complexity, size, purity or the like of a cDNA library is suitable for the desired screening method. In particular, the cDNA library can be processed to make the sample suitable for any high-throughout screening methods, such as Applied Biosystems' SOLiD sequencing technology, or Illumina's Genome Analyzer. As such, the cDNA library can be processed by fragmenting the cDNA library (e.g. with DNase) to obtain a short-fragment 5'-end library. Adapters can be added to the cDNA, e.g. at one or both ends to facilitate sequencing of the library. The cDNA library can be further amplified, e.g. by PCR, to obtain a sufficient quantity of cDNA for sequencing.

Embodiments of the invention provide a cDNA library produced by any of the methods described herein. This cDNA library can be sequenced to provide an analysis of gene expression in single cells or in a plurality of single cells.

Embodiments of the invention also provide a method for analyzing gene expression in a plurality of single cells, the method comprising the steps of preparing a cDNA library using the method described herein and sequencing the cDNA library. A "gene" refers to a polynucleotide containing at least one open reading frame (ORF) that is capable of encoding a particular polypeptide or protein after being transcribed and translated. Any of the polynucleotide sequences described herein can be used to identify larger fragments or full-length coding sequences of the gene with which they are associated. Methods of isolating larger fragment sequences are known to those of skill in the art.

As used herein, "expression" refers to the process by which polynucleotides are transcribed into mRNA and/or the process by which the transcribed mRNA is subsequently being translated into peptides, polypeptides, or proteins. If the polynucleotide is derived from genomic DNA, expression can include splicing of the mRNA in an eukaryotic cell.

The cDNA library can be sequenced by any suitable screening method. In particular, the cDNA library can be sequenced using a high-throughout screening method, such as Applied Biosystems' SOLiD sequencing technology, or Illumina's Genome Analyzer. In one aspect of the invention, the cDNA library can be shotgun sequenced. The number of reads can be at least 10,000, at least 1 million, at least 10 million, at least 100 million, or at least 1000 million. In another aspect, the number of reads can be from 10,000 to 100,000, or alternatively from 100,000 to 1 million, or alternatively from 1 million to 10 million, or alternatively from 10 million to 100 million, or alternatively from 100 million to 1000 million. A "read" is a length of continuous nucleic acid sequence obtained by a sequencing reaction.

"Shotgun sequencing" refers to a method used to sequence very large amount of DNA (such as the entire genome). In this method, the DNA to be sequenced is first shredded into smaller fragments which can be sequenced individually. The sequences of these fragments are then reassembled into their original order based on their overlapping sequences, thus yielding a complete sequence. "Shredding" of the DNA can be done using a number of difference techniques including restriction enzyme digestion or mechanical shearing. Overlapping sequences are typically aligned by a computer suitably programmed. Methods and programs for shotgun sequencing a cDNA library are well know in the art.

An embodiment of the method of the invention is summarized in **Figure 1****.** Cells are obtained from a tissue of interest and a single-cell suspension is obtained. A single cell is placed in one well of a 96-well plate in Cell Capture Mix. The cells are lysed and reverse transcription reaction mix is added directly to the lysates without additional purification. This results in the synthesis of cDNA from cellular mRNA and incorporation of a tag into the cDNA. The tagged cDNA samples are pooled and amplified and then sequenced to produce 100 million reads. This allows identification of genes that are expressed in each single cell.

It is understood that modifications which do not substantially affect the activity of the various embodiments of this invention are also provided within the definition of the invention provided herein. Accordingly, the following examples are intended to illustrate but not limit the present invention.

### EXAMPLE I

### Single-cell tagged reverse transcription (STRT)

An embodiment of the method of the invention may be called "single-cell tagged reverse transcription" (STRT) and is described in detail below.

### Cell Collection and Lysis

A 96-well plate containing Cell Capture Mix was made by aliquoting 5 µl/well from the Cell Capture Master Plate (see Table 1 below) into an AbGene Thermo-Fast plate.

**Table 1 Making a STRT Cell Capture Master Plate.**

| **Reagent** | **For one well** | **For one plate** | **Final concentration** |
|---|---|---|---|
| STRT-T30-BIO (100 µM) | 0.25 µL | 27.5 µL | 400 nM |

| **Reagent** | **For one well** | **For one plate** | **Final concentration** |
|---|---|---|---|
| STRT buffer (5x) | 12.5 µL | 1375 µL | 1x |
| STRT-FW-n (5 µM) | 5 µL | (5 µL/well) | 400 nM |
| Water | 44.75 | 4.9 mL | |
| Total | **62.5 uL** | | |

27.5 µL STRT-T30- BIO (100 µM) was mixed with 1375 µL STRT 5x buffer and 4.9 mL Rnase/Dnase-free water. 57.5 µL of this solution was aliquoted to each well of a 96-well plate and 5 µL/well of STRT-FW-n (from 5 µM stock plate) was added, i.e. a different oligo in each well.

The sequence of STRT-T30-BIO (which is a CDS) is:
5'-BIO-AAGCAGTGGTATCAACGCAGAGT₃₀VN-3',
and the sequence of STRT-FW-n (which is a TSO) is:
5'-AAGCAGTGGTATCAACGCAGAGTGGATGCTXXXXXrGrGrG-3'(X=cell tag)

### BtsCl->2/0

n is 1-96 and each oligonucleotide has a distinct cell tag, such that a different oligonucleotide is added to each well containing a single cell.

Mouse embryonic stem cells (R1) were grown without feeder cells, trypsinized, cleared through cell strainer and resuspended in 1x PBS. Cells were then picked by FACS into the Capture Plate, with a single cell being placed in each well. The Capture Plate was transferred to a PCR thermocycler and incubated at 72°C for 2 minutes, and then cooled to 4°C for 5 minutes to allow annealing to occur. The detergent in STRT buffer helps reduce adsorption of mRNA and cDNA to the walls of the reaction tube during subsequent steps, and also improves lysis of the cells. The heating step causes the cell to lyse completely and release its RNA. When the temperature is reduced, the oligo(dT) primer anneals.

### Reverse transcription

5 µL/well of RT mix (see Table 2 below) was added and the plate was incubated at 42°C for 45 minutes, without heated lid.

**Table 2 Composition of RT mix**

| **Reagent** | **For 96 reactions** | **For one reaction** | **Final conc.** |
|---|---|---|---|
| STRT buffer (5x) | 110 µL | 1 µL | 1x |
| DTT (20 mM) | 110 µL | 1 µL | 2 mM |
| dNTP (10 mM) | 110 µL | 1 µL | 1 mM |
| Dnase/Rnase-free water | 209 µL | 1 µL | |
| Superscript II RT (200 U/µL) | 11 µL | 1 µL of 20 U/µL (dilute in 1x RT buffer) | 2.5 U/µL |
| **Total volume** | **550 µL** | **5 µL** | |

When the RT mix is added, the reverse transcriptase enzyme (Superscript II RT) synthesizes a first strand and the tagged template-switching oligo introduces an upstream primer sequence.

**Figure 2** shows the synthesis of cDNA by template switching. The 5' end of the cDNA (which corresponds to the 3' end of the mRNA) can be controlled by adding a tail (which is oligo-dT in this case) to the cDNA synthesis primer (CDS). The 3' end of the cDNA can be controlled using the template-switching oligo (TSO). When reverse transcriptase reaches the 5' end of the mRNA template, it preferentially adds 2-5 cytosines. The template-switching oligo, which has 2-5 guanosines, anneals transiently, and reverse transcriptase then switches template and synthesizes the complementary strand. By this mechanism, both ends of the cDNA can be arbitrarily controlled.

The structure of a typical TSO is shown in **Figure 3****.** In this particular TSO, the 3' template switching sequence includes three riboguanines (rG). The cell-tag is shown as "XXXXX" and can in general have any length or nucleotide composition. Arbitrary sequence can be inserted at the 5' end of the TSO, after the 5' APS, or after the cell-tag, but not at the 3' end.

The structure of a typical CDS is shown in **Figure 4****.** The RCS is oligo-dT with an anchor nucleotide (V = A,C,G degenerate). The cell tag "XXXXX" can have any length or nucleotide composition. Additional arbitrary sequences can be inserted at the 5' end, after the 5' APS, or after the cell-tag.

### Purification of cDNA

50 µl PBI (Qiaquick PCR Purification Kit) was added to each well to inactivate reverse transcriptase. The PBI inactivates reverse transcriptase and cDNA from all the wells was then pooled. Adding PBI before pooling prevents cDNA synthesis from proceeding once the cDNA samples have been pooled. The pooled cDNA was loaded on a single Qiaquick column and the purified cDNA was eluted in 30 µl EB buffer into a Beckman Polyallomer tube. The purification step removes primers (<40 bp) as well as proteins and other debris.

### Full length cDNA amplification

The cDNA was amplified by PCR by adding the reagents shown in Table 3.

**Table 3. Reagents used for full-length cDNA amplification.**

| **Reagent** | **For one tube:** | **Final conc.** |
|---|---|---|
| Rnase/Dnase-free water | 54 µL | |
| Advantage2 PCR buffer (10x) | 10 µL | 1x |
| dNTP (10 mM) | 2 µL | 200 µM |
| STRT-PCR (10 µM) | 2 µL | 200 µM |
| Advantage2 DNA Polymerase mix | 2 µL | 1x |
| (50x) | | |
| **Total volume** | **100 µL** | |

The sequence of STRT-PCR is:
5'-BIO-AAGCAGTGGTATCAACGCAGAGT-3'

PCR was performed using a heated lid as follows: 1 min @ 95°C, 25 cycles of [5 s @ 95°C, 5 s @ 65°C, 6 min @ 68°C], 4°C forever.

30 µL of the reaction was transferred to a new PCR tube, labeled "Optimization". The remaining 70 µL was stored at 4°C until later. 10 µL from the Optimization tube was removed and the rest of the sample was run for three more cycles. This was repeated to obtain aliquots from 25, 28 and 31 cycles. A diagnostic 2% agarose gel was used to determine the optimal cycle number (which is the cycle just before saturation of the PCR), as well as to visualize the size range of the product (see **Figure 5****).** Typically, the optimal number of cycles was around 28. The remaining 70 µL of reaction was run to reach the optimal number of cycles (in addition to the 25 cycles already run).

The PCR product was purified using a Qiaquick column (PCR purification kit) and eluted in 50 µL EB into a Beckman polyallomer tube. The expected concentration at this stage was about 20-40 ng/µL (1-2 µg total yield).

### DNase treatment

The sample was treated with DNaseI in the presence of Mn²⁺ to generate double-strand breaks and reduce the size. First, the following components were mixed in the order shown in Table 4.

**Table 4. Composition of reaction mix for DNase treatment.**

| **Reagent** | **Volume** | **Final concentration** |
|---|---|---|
| cDNA template | 50 µl | 8 - 16 ng/µL |
| Water | 42.8 µl | |
| 10 x DNase I buffer | 11.6 µl | |
| 100 mM MnCl₂ (*) | 11.6 µl | 10 mM |
| **Total volume** | **116 µl** | |

| | | |
|---|---|---|
| (*) It is crucial to add MnCl₂ last to the reaction, as the BSA present in the buffer will precipitate otherwise. | | |

Diluted DNaseI (0.01 units/µl) was prepared just before use as follows: 40 µL 10x DNase I buffer, 318 µL water, 40 µL 100 mM MnCl₂ and 2 µL DNaseI (2 U/µL).

4 µl of this diluted DNase I was added to the reaction mix described in Table 4, and was incubated at RT for exactly 10 minutes. The reaction was then stopped by adding 600 µL PBI.

The sample was purified on a Qiaquick column and eluted in 30 µL EB.

### Bead capture and end repair/nick translation

The fragments were next bound to beads to capture 5' and 3' ends, and then treated with TaqExpress to repair frayed ends and nicks. 30 µL Dynabeads MyOne C1 Streptavidin were washed twice in 2x B&W (Dynal), then added to the Dnase-treated sample, incubated for 10 minutes, and then washed 3x in 1x B&W. About 10% of the sample was bound to the beads (i.e. about 30 - 60 ng), since internal fragments were not biotinylated.

The beads were washed once in 1x TaqExpress buffer and resuspended in the reaction mix shown in Table 5:

**Table 5. Composition of the reaction mix used for end repair/nick translation.**

| **Reagent** | **Volume** | **Final concentration** |
|---|---|---|
| 10x TaqExpress buffer (blue, with MgCl₂) | 4 µL | 1x |
| dNTP (10 mM) | 0.8 µL | 200 µM |
| Water | 33 µl | |
| TaqExpress (5 U/µL) | 2 µL | 0.25 U/µL |
| **Total volume** | **40 µl** | |

The reaction was incubated at 37°C for 30 minutes, and then washed three times in 1x NEB4 buffer.

### Fragment release and RDV/FDV adapter ligation

The fragments were released by BtsCI digestion, and simultaneously ligated to the FDV and RDV adapters. The beads were then resuspended in the reaction mix shown in Table 6.

**Table 6. Reaction mix for resuspension of the beads.**

| **Reagent** | **Volume** | **Final concentration** |
|---|---|---|
| 10x NEB4 buffer | 4 µL | 1x |
| ATP (10 mM) | 4 µL | 1 mM |
| Adapter STRT-RDV-A (10 µM) | 4 µL | 1 µM |
| Adapter STRT-FDV (10 µM) | 4 µL | 1 µM |
| Water | 26 µL | |
| T4 DNA Ligase (5 U/µL; Invitrogen) | 2 µL | 0.25 U/µL |
| BtsCI (20 U/µL) | 2 µL | 1 U/µL |
| **Total volume** | **40 µl** | |

The sequence of STRT-FDV, made by annealing STRT-ADP1U and STRT-ADP1L, was:
5'------CCACTACGCCTCCGCTTTCCTCTCTATGGGCAGTCGGTGATCT-3'
3'-PHO-GGTGATGCGGAGGCGAAAGGAGAGATACCCGTCAGCCACTA-PHO-5'

The sequence of STRT-RDV-A, made by annealing STRT-ADP2U-T and STRT-ADP2L was:
5'----- AACTGCCCCGGGTTCCTCATTCTCTT-3'
3-PHO-TTGACGGGGCCCAAGGAGTAAGAGA-PHO-5'

The beads were incubated for 30 min at 37°C. The reaction was stopped by adding 200 µL PBI, while the beads were held on the magnet. The supernatant was loaded on a Qiaquick column, purified and eluted in 30 µl EB in a Beckman polyallomer tube. The concentration of the cDNA was about 1 - 2 ng/µL.

### Library PCR amplification

Eight reactions were set up using 4, 2, 1, 1/2, 1/4, 1/8, 1/16 and 1/32 µL aliquots of the adapted library, each in 4 µL. Each library was amplified using the PCR reaction mix shown in Table 7.

**Table 7. PCR reaction mix for cDNA library amplification.**

| **Reagent** | **For one tube:** | **Final conc.** |
|---|---|---|
| cDNA library (dilution series) | 4 µL | (20 pg/µL) |
| TaqExpress blue buffer (10x) | 5 µL | 1x |
| dNTP (10 mN) | 1 µL | 200 µM |
| SOLID-PI (10 µM) | 2 µL | 400 nM |
| SOLID-P2 (10 µM) | 2 µL | 400 nM |

| **Reagent** | **For one tube:** | **Final conc.** |
|---|---|---|
| Rnase/Dnase-free water | 35.7 µL | 0.25 U/µL |
| TaqExpress polymerase (25 U/µL) | 0.3 µL | 0.15 U/µL |
| **Total volume** | **50 µl** | |

The sequence of SOLID-P1 was:
5'-CCACTACGCCTCCGCTTTCCTCTCTATG-3'

The sequence of SOLID-P2 was:
5'-CTGCCCCGGGTTCCTCATTCTCT-3'

PCR was run with heated lid: 5 min @ 94°C, 18 cycles of [15 s @ 94°C, 15 s @ 68°C], 5 min @ 70°C.

All eight reactions were loaded on a 2% E-gel, 10 µL + 10 µL water to determine which reaction was just shy of saturated (see **Figure 6****).**

A fresh PCR reaction was then performed using the optimal number of cycles and starting material. For example, if 1/4 µL was optimal at 18 cycles, then 14 cycles were performed.

The PCR product was loaded on a 2% E-gel, 125 - 200 bp region was excised from the gel and purified by Qiagen Gel Extraction Kit (see **Figure 7****).** The purified cDNA was eluted in 50 µL EB.

The cDNA library was now prepared for SOLiD sequencing, and could go directly into emulsion PCR.

To verify cDNA library quality, an aliquot was cloned using Invitrogen TOPO TA cloning kit, and sequenced by Sanger sequencing. **Figure 8** shows a typical result demonstrating the presence of primer sequences for SOLiD (P1 and P2; underlined), the cell-specific tag (boxed), and the 2-5 Gs (shaded in a gray box) from the template-switching mechanism. From 22 Sanger sequences, 7 were not mappable to anything in GenBank. All except one of these were misligations of the SOLiD adapters, which can be re-designed to stop this happening. In separate experiments, we found no misligated adapters after blocking their blunt ends with 3' phosphate. Alternatively, the non-ligating 3' ends could be blocked using dideoxy nucleotides or by designing a protruding strand incompatible with the ligating ends of the adapters.

Of the remaining 15 sequences, one was a ribosomal RNA (45S), which is not polyadenylated. It probably occurred due to internal mispriming during first-strand synthesis. The remaining 14 reads were all from polyadenylated mRNA, in the correct orientation and with correct cell tags.

To summarize this dataset, 15 of 22 reads were mappable and 14 of these 15 were correct transcript tags. All the transcripts seen in the Sanger sequence dataset are listed below:

| **Gene** | **Length (of mRNA)** |
|---|---|
| Ribosomal protein L35 | 452 |
| B2_Mm2 | ∼200 |
| Tubulin beta 2c | 1 561 |
| B2_Mm1 | ∼195 |
| RIKEN 1110008L16 gene | 3 127 |
| Sod2 | 661 |
| Chchd2 | 910 |
| mt-Cox2 | 947 |
| Hnrnpab | 2 545 |
| Ribosomal protein L24 | 558 |
| Ribosomal protein S18 | 524 |
| RIKEN 2700060E02 | 941 |
| B2_Mm1 | ∼195 |
| Ribosomal protein S28 | 356 |

As expected, the list was dominated by highly expressed genes like ribosomal proteins. Several long transcripts were present in this sample, indicating that there was no strong bias (if any) towards short mRNAs.

Interestingly, three copies of B2 repeats (of subfamilies Mm1 and Mm2) were observed. These are SINE-family repeats expressed from a pol III promoter (not pol II as most mRNAs), but with strong polyadenylation signals. They have been shown to be expressed at extremely high levels in ES cells, together comprising more than 10% of all mRNA. Even more interestingly, they peak just before S-phase in dividing cells, and are thus an early indication that it will be possible to characterize the cell cycle in unsynchronized primary cells using this method.

### Quality control by quantitative real-time PCR

To verify that the libraries were representative of the mRNA content of the original ES cell population, quantitative real-time PCR was performed against a set of markers for pluripotency, as well as markers for differentiated tissues. A cDNA library prepared according to classical methods from 1 µg total RNA (∼100,000 cells) was compared with the library prepared from 96 single cells using the STRT protocol.

Well-known markers of pluripotency, such as Sox2, Oct4 and Nanog were detected at similar levels in both samples, whereas markers of germ layer differentiation such as Brachyury, Gata4 and Eomes were detected only at very low levels in both samples (see **Figure 9****).** The quantitative correlation was good (Pearson correlation coefficient 0.84), with the exception of Plk1, which was not detected in single cells in this experiment.

### Reagents used

**Table 8. List of reagents used in the method described above.**

| **Reagent** | **Source** |
|---|---|
| 2x B&W | 10 mM Tris HCl pH 7.5, 1 mM EDTA, 2 M NaCl |
| 10% Tween-20 stock | Make 10% Tween-20, filter at 0.45 µm |
| Dynabeads MyOne Streptavidin Cl | Invitrogen |
| 5x STRT buffer | 100 mM Tris-HCl pH 8, 375 mM KCl, 30 mM MgCl₂, 0.1% Tween-20 |
| DNase/RNase-free water | Ambion |
| 20 mM DTT | |
| 10 mM and 1 mM dNTP | NEB / In Vitro |
| Superscript II RT | Invitrogen |
| 10x Advantage2 PCR buffer | Clontech |
| 50x Advantage2 polymerase mix | Clontech |
| 10x DNase I buffer | 0.5 M Tris pH 7.5, 0.5 mg/ml BSA |
| 100 mM MnCl₂ | Sigma 1M stock |
| Thermo-Fast 96-well plate | AbGene |
| Thermo-Strip tube strips | AbGene |
| 1.5 mL polyallomer tubes | Beckman-Coulter |
| 2% E-gel with SYBR Safe | Invitrogen |
| Qiaquick PCR Purification Kit | Qiagen |
| Qiaquick Gel Extraction kit | Qiagen |
| BtsCI restriction endonuclease | NEB |
| T4 DNA Ligase | Invitrogen |
| 10 mM ATP | |
| 10x NEB4 buffer | NEB |
| 5 U/µL TaqExpress polymerase | Genetix, UK |
| 10x TaqExpress buffer (blue) | Genetix, UK |

### EXAMPLE II

### Characterization of single-cell transcriptional landscape by highly multiplex RNA-Seq

Understanding of the development and maintenance of tissues has been greatly aided by large-scale gene expression analysis. However, tissues are invariably complex, consisting of multiple cell types in a diversity of molecular states. As a result, expression analysis of a tissue confounds the true expression patterns of its constituent cell types. Described herein is a novel strategy, termed shotgun single-cell expression profiling, was used to access such complex samples. It is a simple and highly multiplexed method used to generate hundreds of single-cell RNA-Seq expression profiles. Cells are then clustered based on their expression profiles, forming a two-dimensional cell map onto which expression data can be projected. The resulting cell map integrates three levels of organization: the whole population of cells, the functionally distinct subpopulations it contains, and the single cells themselves-all without need for known markers to classify cell types. The feasibility of the strategy is demonstrated by analyzing the complete transcriptomes of 436 single cells of three distinct types. This strategy enables the unbiased discovery and analysis of naturally occurring cell types during development, adult physiology and disease.

### METHODS

### Cell culture

ES R1 cells were cultured as previously described (Moliner et al., Stem Cells Dev. 17:233-243 (2008)). MEFs and Neuro-2A cells were grown in DMEM with 10% FBS, 1x penicillin/streptomycin, 1x Glutamax and 0.05 mM 2-mercaptoethanol. All culture reagents were from Gibco.

### Quantitative real-time PCR (Q-PCR)

RNA was isolated using Trizol (Invitrogen) and 1 µg total RNA was reverse transcribed with Superscript III (Invitrogen) and oligo-(dT) primer. SYBR Green Master Mix (Applied Biosystems) and a cDNA amount corresponding to 5 ng RNA was mixed with 4 pmol primers (Eurofins MWG Operon, Germany) in a total volume of 10 µL, and analyzed on a 7900HT real-time thermocycler (Applied Biosystems). A dilution series of the template was used to determine primer efficiency.

### Single-cell tagged reverse transcription (STRT)

Cells were dissociated enzymatically using TrypLE Express (Invitrogen), washed and resuspended in phosphate-buffered saline (PBS). A single cell was collected into each well of a 96-well capture plate (AbGene Thermo-Fast 96 cat. No. 0600) by fluorescence-activated cell sorting (FACS), and the plate was immediately frozen on dry ice. The FACS was used only to collect single cells and to reject dead cells and debris based on light scattering; no fluorescent reporter was used, and hence the collected cells would represent a random sample of the population.

The cell capture plate contained a single cell per well in 5 µL of *STRT* buffer (20 mM Tris-HCl pH 8.0, 75 mM KCl, 6 mM MgCl², 0.02% Tween-20) with 400 nM STRT-T30-BIO (5'-biotin-AAGCAGTGGTATCAACGCAGAGT₃₀VN-3'; this and all other oligos were from Eurofins MWG Operon) and 400 nM STRT-FW-n (5'-AAGCAGTGGTATCAACGCAGAGTGGATGCTXXXXXrGrGrG-3', where "rG" denotes a ribonucleotide guanine and "XXXXX" was a barcode). Each well of the capture plate contained a different template-switching helper oligo (STRT-FW-n) with a distinct barcode. For example, well A01 received STRT-FW-1 with sequence 5'-AAGCAGTGGTATCAACGCAGAGTGGATGCTCAGAArGrGrG-3' having barcode sequence CAGAA. All 96 barcodes and helper oligo sequences are given in Table 9.

**Table 9. Barcodes and helper oligonucleotide sequences.**

| **Barcode** | **Oligo name** | **Sequence (GGG) = ribonucleotides** |
|---|---|---|
| CAGAA | STRT-FW-1 | AAGCAGTGGTATCAACGCAGAGTGGATGCTCAGAA(GGG) |
| CATAC | STRT-FW-2 | AAGCAGTGGTATCAACGCAGAGTGGATGCTCATAC(GGG) |
| CAAAG | STRT-FW-3 | AAGCAGTGGTATCAACGCAGAGTGGATGCTCAAAG(GGG) |
| CACAT | STRT-FW-4 | AAGCAGTGGTATCAACGCAGAGTGGATGCTCACAT(GGG) |
| CATCA | STRT-FW-5 | AAGCAGTGGTATCAACGCAGAGTGGATGCTCATCA(GGG) |
| CAGCC | STRT-FW-6 | AAGCAGTGGTATCAACGCAGAGTGGATGCTCAGCC(GGG) |
| CACCG | STRT-FW-7 | AAGCAGTGGTATCAACGCAGAGTGGATGCTCACCG(GGG) |
| CAACT | STRT-FW-8 | AAGCAGTGGTATCAACCCAGAGTGGATGCTCAACT(GGG) |
| CAAGA | STRT-FW-9 | AAGCAGTGGTATCAACGCAGAGTGGATGCTCAAGA(GGG) |
| CACGC | STRT-FW-10 | AAGCAGTGGTATCAACGCAGAGTGGATGCTCACGC(GGG) |
| CATGT | STRT-FW-11 | AAGCAGTGGTATCAACGCAGAGTGGATGCTCATGT(GGG) |
| CACTA | STRT-FW-12 | AAGCAGTGGTATCAACGCAGAGTGGATGCTCACTA(GGG) |
| CAATC | STRT-FW-13 | AAGCAGTGGTATCAACGCAGAGTGGATGCTCAATC(GGG) |
| CATTG | STRT-FW-14 | AAGCAGTGGTATCAACGCAGAGTGGATGCTCATTG(GGG) |
| CAGTT | STRT-FW-15 | AAGCAGTGGTATCAACGCAGAGTGGATGCTCAGTT(GGG) |
| CCTAA | STRT-FW-16 | AAGCAGTGGTATCAACGCAGAGTGGATGCTCCTAA(GGG) |
| CCGAC | STRT-FW-17 | AAGCAGTGGTATCAACGCAGAGTGGATGCTCCGAC(GGG) |
| CCAAT | STRT-FW-18 | AAGCAGTGGTATCAACGCAGAGTGGATGCTCCAAT(GGG) |
| CCGCA | STRT-FW-19 | AAGCAGTGGTATCAACGCAGAGTGGATGCTCCGCA(GGG) |
| CCTCC | STRT-FW-20 | AAGCAGTGGTATCAACGCAGAGTGGATGCTCCTCC(GGG) |
| CCACG | STRT-FW-21 | AAGCAGTGGTATCAACGCAGAGTGGATGCTCCACG(GGG) |
| CCAGC | STRT-FW-22 | AAGCAGTGGTATCAACGCAGAGTGGATGCTCCAGC(GGG) |
| CCTGG | STRT-FW-23 | AAGCAGTGGTATCAACGCAGAGTGGATGCTCCTGG(GGG) |
| CCGGT | STRT-FW-24 | AAGCAGTGGTATCAACGCAGAGTGGATGCTCCGGT(GGG) |
| CCATA | STRT-FW-25 | AAGCAGTGGTATCAACGCAGAGTGGATGCTCCATA(GGG) |
| CCGTG | STRT-FW-26 | AAGCAGTGGTATCAACGCAGAGTGGATGCTCCGTG(GGG) |
| CCTTT | STRT-FW-27 | AAGCAGTGGTATCAACGCAGAGTGGATGCTCCTTT(GGG) |
| CGAAA | STRT-FW-28 | AAGCAGTGGTATCAACGCAGAGTGGATGCTCGAAA(GGG) |
| CGCAC | STRT-FW-29 | AAGCAGTGGTATCAACGCAGAGTGGATGCTCGCAC(GGG) |
| CGGAG | STRT-FW-30 | AAGCAGTGGTATCAACGCAGAGTGGATGCTCGGAG(GGG) |
| CGTAT | STRT-FW-31 | AAGCAGTGGTATCAACGCAGAGTGGATGCTCGTAT(GGG) |
| CGCCA | STRT-FW-32 | AAGCAGTGGTATCAACGCAGAGTGGATGCTCGCCA(GGG) |
| CGACC | STRT-FW-33 | AAGCAGTGGTATCAACGCAGAGTGGATGCTCGACC(GGG) |
| CGTCG | STRT-FW-34 | AAGCAGTGGTATCAACGCAGAGTGGATGCTCGTCG(GGG) |
| CGGCT | STRT-FW-35 | AAGCAGTGGTATCAACGCAGAGTGGATGCTCGGCT(GGG) |
| CGGGA | STRT-FW-36 | AAGCAGTGGTATCAACGCAGAGTGGATGCTCGGGA(GGG) |
| CGTGC | STRT-FW-37 | AAGCAGTGGTATCAACGCAGAGTGGATGCTCGTGC(GGG) |
| CGAGG | STRT-FW-38 | AAGCAGTGGTATCAACGCAGAGTGGATGCTCGAGG(GGG) |
| CGCGT | STRT-FW-39 | AAGCAGTGGTATCAACGCAGAGTGGATGCTCGCGT(GGG) |
| CGTTA | STRT-FW-40 | AAGCAGTGGTATCAACGCAGAGTGGATGCTCGTTA(GGG) |
| CGGTC | STRT-FW-41 | AAGCAGTGGTATCAACGCAGAGTGGATGCTCGGTC(GGG) |
| CGCTG | STRT-FW-42 | AAGCAGTGGTATCAACGCAGAGTGGATGCTCGCTG(GGG) |
| CGATT | STRT-FW-43 | AAGCAGTGGTATCAACGCAGAGTGGATGCTCGATT(GGG) |
| CTCAA | STRT-FW-44 | AAGCAGTGGTATCAACGCAGAGTGGATGCTCTCAA(GGG) |
| CTAAC | STRT-FW-45 | AAGCAGTGGTATCAACGCAGAGTGGATGCTCTAAC(GGG) |
| CTTAG | STRT-FW-46 | AAGCAGTGGTATCAACGCAGAGTGGATGCTCTTAG(GGG) |
| CTGAT | STRT-FW-47 | AAGCAGTGGTATCAACGCAGAGTGGATGCTCTGAT(GGG) |
| CTACA | STRT-FW-48 | AAGCAGTGGTATCAACGCAGAGTGGATGCTCTACA(GGG) |
| CTGCG | STRT-FW-49 | AAGCAGTGGTATCAACGCAGAGTGGATGCTCTGCG(GGG) |
| CTTCT | STRT-FW-50 | AAGCAGTGGTATCAACGCAGAGTGGATGCTCTTCT(GGG) |
| CTTGA | STRT-FW-51 | AAGCAGTGGTATCAACGCAGAGTGGATGCTCTTGA(GGG) |
| CTGGC | STRT-FW-52 | AAGCAGTGGTATCAACGCAGAGTGGATGCTCTGGC(GGG) |
| CTCGG | STRT-FW-53 | AAGCAGTGGTATCAACGCAGAGTGGATGCTCTCGG(GGG) |
| CTAGT | STRT-FW-54 | AAGCAGTGGTATCAACGCAGAGTGGATGCTCTAGT(GGG) |
| CTGTA | STRT-FW-55 | AAGCAGTGGTATCAACGCAGAGTGGATGCTCTGTA(GGG) |
| CTTTC | STRT-FW-56 | AAGCAGTGGTATCAACGCAGAGTGGATGCTCTTTC(GGG) |
| CTATG | STRT-FW-57 | AAGCAGTGGTATCAACGCAGAGTGGATGCTCTATG(GGG) |
| CTCTT | STRT-FW-58 | AAGCAGTGGTATCAACGCAGAGTGGATGCTCTCTT(GGG) |
| GACAA | STRT-FW-59 | AAGCAGTGGTATCAACGCAGAGTGGATGCTGACAA(GGG) |
| GAAAC | STRT-FW-60 | AACCAGTGGTATCAACGCAGAGTGGATGCTGAAAC(GGG) |
| GATAG | STRT-FW-61 | AAGCAGTGGTATCAACGCAGAGTGGATGCTGATAG(GGG) |
| GAGAT | STRT-FW-62 | AAGCAGTGGTATCAACGCAGAGTGGATGCTGAGAT(GGG) |
| GAACA | STRT-FW-63 | AAGCAGTGGTATCAACGCAGAGTGGATGCTGAACA(GGG) |
| GAGCG | STRT-FW-64 | AAGCAGTGGTATCAACGCAGAGTGGATGCTGAGCG(GGG) |
| GATCT | STRT-FW-65 | AAGCAGTGGTATCAACGCAGAGTGGATGCTGATCT(GGG) |
| GATGA | STRT-FW-66 | AAGCAGTGGTATCAACGCAGAGTGGATGCTGATGA(GGG) |
| GAGGC | STRT-FW-67 | AAGCAGTGGTATCAACGCAGAGTGGATGCTGAGGC(GGG) |
| GACGG | STRT-FW-68 | AAGCAGTGGTATCAACGCAGAGTGGATGCTGACGG(GGG) |
| GAAGT | STRT-FW-69 | AAGCAGTGGTATCAACGCAGAGTGGATGCTGAAGT(GGG) |
| GAGTA | STRT-FW-70 | AAGCAGTGGTATCAACGCAGAGTGGATGCTGAGTA(GGG) |
| GATTC | STRT-FW-71 | AAGCAGTGGTATCAACGCAGAGTGGATGCTGATTC(GGG) |
| GAATG | STRT-FW-72 | AAGCAGTGGTATCAACGCAGAGTGGATGCTGAATG(GGG) |
| GACTT | STRT-FW-73 | AAGCAGTGGTATCAACGCAGAGTGGATGCTGACTT(GGG) |
| GCAAA | STRT-FW-74 | AAGCAGTGGTATCAACGCAGAGTGGATGCTGCAAA(GGG) |
| GCCAC | STRT-FW-75 | AAGCAGTGGTATCAACGCAGAGTGGATGCTGCCAC(GGG) |
| GCGAG | STRT-FW-76 | AAGCAGTGGTATCAACGCAGAGTGGATGCTGCGAG(GGG) |
| GCTAT | STRT-FW-77 | AAGCAGTGGTATCAACGCAGAGTGGATGCTGCTAT(GGG) |
| GCACC | STRT-FW-78 | AAGCAGTGGTATCAACGCAGAGTGGATGCTGCACC(GGG) |
| GCTCG | STRT-FW-79 | AAGCAGTGGTATCAACGCAGAGTGGATGCTGCTCG(GGG) |
| GCGCT | STRT-FW-80 | AAGCAGTGGTATCAACGCAGAGTGGATGCTGCGCT(GGG) |
| GCGGA | STRT-FW-81 | AAGCAGTGGTATCAACGCAGAGTGGATGCTGCGGA(GGG) |
| GCTGC | STRT-FW-82 | AAGCAGTGGTATCAACGCAGAGTGGATGCTGCTGC(GGG) |
| GCAGG | STRT-FW-83 | AAGCAGTGGTATCAACGCAGAGTGGATGCTGCAGG(GGG) |
| GCCGT | STRT-FW-84 | AAGCAGTGGTATCAACGCAGAGTGGATGCTGCCGT(GGG) |
| GCTTA | STRT-FW-85 | AAGCAGTGGTATCAACGCAGAGTGGATGCTGCTTA(GGG) |
| GCGTC | STRT-FW-86 | AAGCAGTGGTATCAACGCAGAGTGGATGCTGCGTC(GGG) |
| GCCTG | STRT-FW-87 | AAGCAGTGGTATCAACGCAGAGTGGATGCTGCCTG(GGG) |
| GCATT | STRT-FW-88 | AAGCAGTGGTATCAACGCAGAGTGGATGCTGCATT(GGG) |
| GGTAA | STRT-FW-89 | AAGCAGTGGTATCAACGCAGAGTGGATGCTGGTAA(GGG) |
| GGCAG | STRT-FW-90 | AAGCAGTGGTATCAACGCAGAGTGGATGCTGGCAG(GGG) |
| GGAAT | STRT-FW-91 | AAGCAGTGGTATCAACGCAGAGTGGATGCTGGAAT(GGG) |
| GGTCC | STRT-FW-92 | AAGCAGTGGTATCAACGCAGAGTGGATGCTGGTCC(GGG) |
| GGACG | STRT-FW-93 | AAGCAGTGGTATCAACGCAGAGTGGATGCTGGACG(GGG) |
| GGCCT | STRT-FW-94 | AAGCAGTGGTATCAACGCAGAGTGGATGCTGGCCT(GGG) |
| GGCGA | STRT-FW-95 | AAGCAGTGGTATCAACGCAGAGTGGATGCTGGCGA(GGG) |
| GGAGC | STRT-FW-96 | AAGCAGTGGTATCAACGCAGAGTGGATCCTGGAGC(GGG) |

The cell capture plate was thawed and then heated to lyse the cells (20°C for 5 minutes, 72°C for 4 minutes, 10°C for 5 minutes in a thermocycler). 5 µL reverse transcription mix (4 mM DTT, 2 mM dNTP, 5 U/µL Superscript II in *STRT* buffer) was added to each well and the plate was incubated (10°C for 10 minutes, 42°C for 45 minutes) to complete reverse transcription and template switching.

To purify the cDNA and remove unreacted primers, 50 µL PB (Qiaquick PCR Purification Kit, Qiagen) was added to each well. All 96 reactions were pooled and purified over a single Qiaquick column. The cDNA was eluted in 30 µL EB in a 1.5 mL polyallomer tube (Beckman).

The whole 96-cell cDNA sample was amplified in a single tube in 100 µL of 200 µM dNTP, 200 µM STRT-PCR primer (5'-biotin-AAGCAGTGGTATCAACGCAGAGT-3'; Eurofins MWG Operon), 1x Advantage2 DNA Polymerase Mix (Clontech) in 1x Advantage2 PCR buffer (Clontech) with 1 min at 94°C followed by 25 cycles of 15 s at 95°C, 30 s at 65°C, 3 min at 68°C, with heated lid. An aliquot was visualized on a 1.2% agarose E-gel (Invitrogen) and the sample was amplified an additional 1-5 cycles if necessary. The product was purified (Qiaquick PCR Purification Kit, Qiagen) and quantified by fluorimetry (Qubit, Invitrogen). Typical yields were 0.5 - 1 µg total. Aliquots were taken at this stage for microarray analysis and Q-PCR.

### Sample preparation for high-throughput sequencing

Amplified cDNA was fragmented by DNase I in the presence of Mn²⁺, which causes a preference for double-strand breaks. 50 µL cDNA was fragmented in DNase I buffer supplemented with 10 mM MnCL² and DNase I diluted to 0.0003 U/µL in a total volume of 120 µL for exactly six minutes room temperature. The reaction was stopped by the addition of 600 µL PB (Qiaquick PCR Purification Kit, Qiagen), purified and eluted in 30 µL EB into a polyallomer tube (Beckman).

3' and 5' fragments were immobilized on 30 µL streptavidin-coated paramagnetic beads (Dynabeads MyOne Cl, Invitrogen), then resuspended in 30 µL TaqExpress buffer (Genetix, UK). Ends were repaired and single A overhangs generated by incubating the beads in 40 µL of 200 µM dNTP, 0.25 U/µl. TaqExpress (Genetix, UK) in TaqExpress buffer at 37°C for 30 minutes, followed by three washes in NEBuffer 4 (New England Biolabs).

5' fragments containing barcodes and cDNA inserts were released from the beads by BtsCl digestion, and adapters were simultaneously ligated to generate a sample suitable for sequencing on the Illumina Genome Analyzer. The beads were resuspended in 40 µL of I mM ATP, 1 µM SOLEXA-ADP1 adapter (5'-AATGATACGGCGACCACCGAGATCTACACTCTTTCCCTACACGACGCTCTTCCG ATCT-3' and 3'-PHO-TTACTATGCCGCTGGTGGCTCTAGATGTGAGAAAGGGATGTGCTGCGAGAAG GCTA-PHO-5'), 1 µM SOLEXA-ADP2 adapter (5'-CAAGCAGAAGACGGCATACGAGCTCTTCCGATCT-3' and 3'-PHO-GTTCGTCTTCTGCCGTATGCTCGAGAAGGCTAG-PHO-5'), 0.25 U/µL T4 DNA ligase (Invitrogen), 1 U/µL BtsCl (New England Biolabs) in 1x NEBuffer 4, and incubated 30 minutes at 37°C. The beads were removed and the supernatant was purified using AmPure (Agencourt) and eluted in 40 µL EB (Qiagen).

The sample was loaded on a 2% SizeSelect E-gel and the range 200 - 300 bp was collected. A 4 µL aliquot was amplified in 50 µL total volume containing 200 µM dNTP, 400 nM each primer (5'- AATGATACGGCGACCACCGA-3' and 5'-CAAGCAGAAGACGGCATACGAG-3') and 0.15 U/µL Phusion polymerase in Phusion HF buffer (New England Biolabs) with 30 s at 98°C, 14-18 cycles of [10 s at 98°C, 30 s at 65°C, 30 s at 72°C] followed by 5 min at 70°C. Test amplifications were used to determine the minimal number of cycles needed. The amplified sample was purified by Qiaquick PCR Purification followed by a 2% SizeSelect E-gel, again collecting the region 200 - 300 bp. The concentration was measured by Qubit (Invitrogen) and was typically 5 ng/µL. Aliquots were cloned (TOPO, Invitrogen) and sequenced by Sanger sequencing to verify sample quality and determine the average fragment length. Based on this information, the molar concentration could be accurately determined and was generally above 10 nM. Cluster formation and sequencing-by-synthesis was performed on a Genome Analyzer *IIx* according to the manufacturer's protocols (Illumina, Inc., San Diego, USA) at a commercial service provider (Fasteris SA, Geneva, Switzerland).

### Mapping, quantification and visualization

Raw reads were sorted by barcode (first five bases) and trimmed to remove up to five 5' Gs introduced by template-switching, and 3' As that sometimes occurred when a read extended into the poly(A) tail. Only exact barcodes were allowed, and the barcodes were designed so that no single error would convert one valid barcode into another. The reads were then mapped to the mouse genome using Bowtie (Langmead et al., Genome Biol. 10:R25 (2009)) with the default settings. Unmapped reads were discarded. Then, for each annotated feature in the NCBI 37.1 assembly, all mapping reads were counted to generate a raw count. That is, all reads that mapped to any of the exons of a gene were assigned to that gene; isoforms were not distinguished. Finally, the raw reads for each cell were normalized to transcripts per million (t.p.m.). Wells with fewer than 1000 mapped reads were omitted from further analysis; presumably these included cases where the FACS instrument had failed to hit the reagent droplet while cells were picked.

To visualize cells in a two-dimensional landscape, all pairwise similarities were first computed. The Bray-Curtis distance was used as a similarity metric because it tended to handle the noise in low-expressed genes well. Standard correlation yielded similar results, but with a few more misplaced cells (data not shown). A similarity graph was then built by letting nodes represent cells, and connecting each cell to its five most similar cells (for clarity, cells with fewer than 10,000 reads were omitted, as they were liable to generate spurious edges). Thus every node (cell) had five outgoing edges and varying numbers of incoming edges. Force-directed layout was then used to project the graph to two dimensions, revealing the internal structure based on cell-cell similarities. The GraphPlot function of the Mathematica program (Wolfram Research Inc., USA) was used with the "SpringElectricalEmbedding" option.

### RESULTS

Data from 436 single cells collected from three different mouse cell types: embryonic stem cells (ES R1, Wood et al., Nature 365: 87-89 (1993)), a neuroblastoma tumor cell line (Neuro-2A, Olmsted et al., Proc. Natl. Acad. Sci U.S.A. 65: 129-136 (1970)) and embryonic fibroblasts (MEF) are reported. In brief, each sample was prepared by picking single cells by fluorescence-activated cell sorting (FACS) into the wells of a 96-well PCR plate preloaded with lysis buffer; heating the plate to complete lysis, then adding reverse transcription reagents to generate a first strand cDNA. To incorporate a well-specific (and hence cell-specific) barcode, the reverse transcriptase template-switching mechanism (Schmidt et al., Nucleic Acids Res. 27:e31 (1999)) was used whereby a helper oligo directs the incorporation of a specific sequence at the 3' end of the cDNA molecule **(****Figure 10A****).** A different helper oligo was used in each well, with distinct five-base barcodes and universal primer sequence. After cDNA synthesis, the 96 reactions were pooled, purified and amplified by single-primer PCR in a single tube. Cell-to-cell amplification bias was thus reduced, and the number of PCR cycles could be kept low since amplification started from 96-fold more material. The amplified samples were then adapted for sequencing using standard methods. The procedure was named STRT' (single-cell tagged reverse transcription). For details, see the Methods section and **Figure 11****.**

5 - 12 million raw reads per sequencing lane were typically obtained on an Illumina Genome Analyzer IIx, and each sample was analyzed on up to eight lanes (but typically one or two). Reads lacking a proper barcode, mostly caused by errors in sample preparation such as misligated adaptors, were removed. Of the remaining 79±11% (mean±s.d.) reads, about three-quarters (75±12%) could be placed on the mouse genome allowing for up to two sequencing errors, resulting in hits to 14,718±3,006 distinct features (including mRNA, mitochondrial RNA and expressed repeats). These results are summarized in Table 10

**Table 10. Library summaries. Percentages refer to percent of the preceding column unless otherwise noted. "Features" includes mRNA, mitochondrial RNA and expressed retrotransposon families. "Cumulative features" tracks the cumulative number of features across all lib**

| ***Library*** | ***Species*** | ***Cell source*** | ***Lanes*** | ***Length (bp)*** | ***Raw reads*** | ***Barcoded*** | ***Mapped*** | ***Features*** | **Cum. features** |
|---|---|---|---|---|---|---|---|---|---|
| L006 | Mouse | ES R1 | 8 | 1x36 | 46,130,389 | 38,284,613 (83%) | 21,356,066 (56%) | 13,168 | 13,168 |
| | | | | 7x50 | | | | | |
| L013 | Mouse | ES R1 | 1 | 36 | 6,482,712 | 5,328,081 (82%) | 3,199,050 (60%) | 19,328 | 21,285 |
| L019A | Mouse | ES R1 | 1 | 36 | 4,302,649 | 2,588,536 (60%) | 2,344,394 (91%) | 12,229 | |
| L019B | Mouse | ES R1 (*) | 1 | 36 | 5,134,171 | 3,323,243 (65%) | 2,562,559 (77%) | 14,794 (**) | 23,152 |
| L023 | Mouse | Neuro2A | 1 | 50 | 10,062,086 | 8,588,048 (86%) | 5,963,143 (69%) | 11,464 | 23,735 |
| L026A | Mouse | MEF | 1 | 36 | 4,278,888 | 3,729,229 (87%) | 2,862,951 (77%) | 12,924 | |
| L026B | Mouse | MEF (*) | 1 | 36 | 3,912,144 | 3,398,519 (87%) | 2,599,074 (76%) | 13,832 (**) | 24,288 |

| | | | | | | | | | |
|---|---|---|---|---|---|---|---|---|---|
| (*) these samples were independently amplified from corresponding 'A' libraries, but were not quality-controlled by Sanger sequencing at preparation (**) cumulative with the previous sample (i.e. A and B together) | | | | | | | | | |

Hits spanned some transcripts (**Figure 10B****),** but were more commonly located in a region approximately 200 - 1500 bp from the 3' end of each gene, as illustrated in **Figure 10C****.** This was expected because an oligo(dT) primer was used to generate cDNA from the 3' end and the mRNA was likely partially degraded during cell lysis by high-temperature hydrolysis in the presence of Mg²⁺. The background from e.g. genomic DNA contamination, judged by hits to unannotated sequence, was minimal (<10⁻³ reads per million per kilobase), as clearly seen in **Figure 10B** and **Figure 10C**. Note the paucity of hits to the reverse strand and to introns for both Pou5f1 and Nanog; other loci were similar.

Scrutinizing the mapped data, no evidence of mispriming or other undesired side-reactions was found. Control experiments showed that both RNA, reverse transcriptase and template switching oligo were individually required to yield product (data not shown). The vast majority of mapped reads had a properly oriented barcode, indicating that they were primed from the oligo-dT primer and correctly template-switched. No evidence of a motif complementary to any of the primers near read mapping sites or indeed of any other motif was found, except for a weak general T-bias in rare cases **(****Figure 12****).** On the other hand, there were frequent hotspots of template switching **(****Figure 13****),** indicating structural constraints in RNA affecting cDNA synthesis and/or template-switching at particular sites. Hotspots were consistent between cells, and should therefore not affect quantitative comparisons.

To characterize sample complexity, and to determine the depth of sequencing required to sample most of the available complexity, the 'new discovery' rate as function of read depth was studied. In other words, the number of new, distinct molecules that were discovered as more sequences were added was determined. It should be noted that, at most, one amplifiable clone was generated from each polyadenylated RNA molecule and this clone was then amplified and sequenced from its 5' end. Therefore reads mapping to distinct locations must have been generated from distinct mRNA molecules. On the other hand, reads mapping to the same location may have been coincidentally generated from two mRNA molecules, or may represent copies of the sample initial clone. The number of distinctly mapping reads was therefore a lower bound on the true sample complexity. As shown in **Figure 14A****,** none of the samples reported here were sequenced to saturation, even at 21 million mapped reads, but the yield of new molecules tended to slow down after about 5-10 million reads. Projecting the curves to infinite read depth, most samples would seem to contain at least three million distinct molecules, or about 30,000 per cell. Assuming 10⁵ - 10⁶ mRNA molecules per cell, this would suggest the method successfully converted 3-30% of the mRNA to mappable reads, as a (very) conservative lower bound. The true proportion was likely significantly higher, as many coincident reads (discarded in this analysis) would be generated from template switching hot spots, as mentioned above.

In contrast, the rate of discovery of distinct features rapidly diminished, and 86% of all distinct feature were detected in the first 14% reads **(****Figure 14B****).** This suggests that the method successfully recovered most expressed features present in the samples, even at relatively low read depth.

Strand information is often required to properly assign reads to transcriptional units, since genes frequently overlap on opposite strands. For example, more than 3000 human genes overlap in this manner (Yelin et al., Nat. Biotechnol. 21:379-386 (2003)). Because the template-switching mechanism used to introduce a barcode occurs directionally, strandedness could be preserved throughout the protocol. To confirm this, the mitochondrial genome was examined, which is expressed as a single long transcript from one strand (the H strand), and is subsequently cleaved to excise tRNA transcripts located between protein-coding genes. Only the protein-coding genes are then polyadenylated. A single protein-coding transcript, ND6, is generated from the L strand, but it is very weakly expressed and irregularly polyadenylated (Slomovic et al., Mol. Cell. Biol. 25:6427-6435 (2005)). As shown in **Figure 10D****,** very strong strand-specificity (>99% of reads on the H strand) was observed and no significant expression of tRNA genes was detected, which confirms that the method was poly(A)-specific. The small number of hits on the L strand occurred mainly near the L strand promoter, which may be explained by the polyadenylation of aborted L-strand transcripts (Slomovic et al., *supra*). Similarly, the apparent expression of ND6 on the wrong strand is likely explained by the natural polyadenylation of ND5 downstream of its open reading frame (Slomovic et al., *supra*). The strand specificity allowed us to unambiguously assign reads to expressed transcripts, even in cases where two overlapping genes were co-expressed **(****Figure 15****).**

On the larger scale of the nuclear chromosomes, hits were approximately equally distributed on the forward and reverse strands. Read density correlated strongly with gene density as shown for chromosome 19 in **Figure 10E****,** again indicating that most reads originated specifically from expressed transcripts and were accurately mapped to the genome.

In order to generate a quantitative measure of gene expression, the number of hits to each annotated feature, normalized to transcripts per million (t.p.m.) were counted. Assuming 10⁵ to 10⁶ transcripts per cell, 1 to 10 t.p.m. corresponds to a single mRNA molecule per cell. Transcript length (as in the RPKM measure (Mortazavi et al., Nat. Methods 5:621-628 (2008))) was not used to normalize because a single amplifiable 3'-end molecule was generated for each input mRNA molecule, irrespective of its length. An advantage of this approach was the lack of bias against short transcripts (which must be sampled more deeply to generate a detectable RPKM value) or long transcripts (which might otherwise be suppressed during PCR). Indeed, and in contrast to standard RNA-Seq (Oshlack et al., Biol. Direct 4:14 (2009)), no length-dependent bias for transcripts longer than 800 nucleotides was observed **(****Figure 16****).** Transcripts shorter than about 200 nucleotides were undercalled, likely because only samples above 100 bp were gel-selected. Additionally, transcripts around 600 nucleotides were slightly overrepresented, possibly because of the higher efficiency of template-switching at the 5'-cap of mRNA (Schmidt et al., Nucleic Acids Res. 27:e31 (1999)) or due to the presence of a few very highly expressed genes in this range (e.g. Dppa5 and Rps14).

Expression levels spanned four orders of magnitude in single cells (approximately 1 - 10,000 t.p.m.), with most genes expressed at low levels (<100 t.p.m.; **Figure 17A****).** Given the relatively shallow depth of sequencing used here, genes expressed below 10 t.p.m. were generally undetectable solely due to the sampling limit. Since single-cell cDNA was pooled before amplification, the yields of different cells could not be subsequently normalized. As a consequence, cells were unequally sampled and the limit of detection varied. For example, compare two cells sampled at 500,000 reads **(****Figure 17B****)** and 100,000 reads **(****Figure 17C****).** In the former case, the apparent detection limit was around 10 t.p.m., whereas in the latter case genes below 100 t.p.m. were generally not detected. However, in both cases, genes above the detection limit were reproducibly quantified in single cells (the coefficient of variation was 46% at 500,000 reads; and 72% at 100,000 reads). Extending this analysis to all cells and genes showed that sensitivity approached the theoretical limit imposed by sampling depth **(****Figure 17D****);** the difference can be explained by losses in reverse transcription, template switching and sample handling. Measured expression levels were generally accurate, as determined by comparison with Q-PCR **(****Figure 17E****),** and microarray hybridization **(****Figure 18****).** In agreement with published reports based on Q-PCR (Bengtsson et al., Genome Res. 15:1388-1392 (2005)), Actb mRNA abundance showed an approximately log-normal distribution across cells **(****Figure 19****).** RNA polymerase II (large subunit) was expressed at 25±123 t.p.m. in ES cells, comparable to the 27 RPKM found by RNA-Seq (Cloonan et al., Nat. Methods 5:613-619 (2008)) and to the 33±79 t.p.m. found in CHO cells by direct detection *in situ* (assuming 300,000 transcripts per cell) (Raj et al., PLoS Biol 4:309 (2006)). The cell-cell relationships on a two-dimensional map were visualized, such that more closely related cells would be located near each other. In this way, cell types based solely on expression data were able to be detected and distinguished, without relying on pre-existing markers. A conventional principal component analysis (PCA) revealed three distinct groups of cells, as expected **(****Figure 20****).** However, a more complete separation into distinct cell-type clusters was achieved using a graph-based method (see **Methods).** Briefly, a graph with nodes representing cells, and edges representing cell-cell similarity of expression pattern was constructed **(****Figure 21A****).** Force-directed layout to project the graph to two dimensions was used. In this test case using only two cell types (ES and Neuro2A cells), a near-perfect separation was achieved **(****Figure 21B****).** A larger map incorporating MEFs and additional ES cells showed good separation **(****Figure 21C****);** demonstrating that the single-cell expression profiles contained enough information to distinguish cell types *de novo.* Both PCA and graph-based analysis clearly distinguished the cell types tested here, but the graph-based method generated more homogenous, well-separated clusters. Both methods accurately grouped independently prepared ES cells together as distinct from the other cell types, showing that clusters did not represent sample preparation artifacts.

Gene expression data was projected onto the map, which provided an easy way to quickly grasp gene expression patterns in both single cells and in the clusters representing cell types **(****Figure 22****).** A set of well-known ES cell markers (Dppa5, Sox2, Sall4, Pou5f1, Nanog, Zfp42, Zic3, Esrrb) were clearly specifically expressed in ES cells, although their expression levels varied widely from cell to cell (note the logarithmic color scale). A few genes important for pluripotency (Klf4, Myc and Klf2) were more broadly expressed. The power of large-scale single-cell analysis was evident in the fact that while not every cell expressed every marker, patterns of gene activity were highly consistent at the cluster level. For example, even a highly expressed cytoskeletal gene like Actb was not always detected, but its expression in each of the three major clusters was obvious. Correspondingly, lower expressed transcription factors characteristic of ES cells went undetected in some individual ES cells, but the overall pattern of expression in the ES cell cluster was unambiguous and consistent with their identity as ES cells. In general, as the average expression levels decreased from 45 000 t.p.m. (mt_Rnr2) to 1700 (Actb), 850 (Rpl4), 73 (Kras) and 0 t.p.m. (Calb1), the number of expressing cells also decreased, reflecting the stochastic nature of gene expression as well as the sensitivity limits of the method.

The cell map representation demonstrated that (1) individual cells showed highly variable expression patterns, yet their overall pattern of expression was sufficient to group cells of one type together as a cluster; (2) once a cluster of cells was formed, representing a distinct cell type, patterns of gene expression (at the cluster level) were unambiguous. Thus, shotgun single-cell expression profiling is an efficient strategy to access single-cell expression data in heterogeneous populations of cells.

### DISCUSSION

Described herein is a reliable and accurate method to obtain RNA-Seq transcription profiles from hundreds of single cells, and shown that single-cell expression profiles can be used to form cell type-specific clusters. This allows analysis of cell type-specific patterns of gene expression both at the single-cell level and the population level, without the need for known markers or even a prior knowledge that a certain cell type exists. This general strategy can be extended to study all kinds of mixed samples. For example, it could be applied to monitor the emergence of specific cell types during organogenesis, without the need to purify those cell types using cell surface markers. Similarly, it could be used to study small populations of stem cells embedded in adult tissues, such as the stem cells that maintain intestinal crypts. The method could also be applied to disease, including the characterization of heterogeneous tumor cell samples or the rare circulating cancer cells that can contribute to metastasis.

What unites all these disparate scientific lines of inquiry is the need to unmix heterogeneous populations of cells. Currently, unmixing is primarily achieved either by physically isolating cells based on known cell surface markers, or by genetically labeling the desired cells so that they can be isolated based on e.g. GFP expression. However, the use of previously known markers precludes the discovery of new cell types, and always risks resulting in mixed data if the markers were not truly specific. In contrast, the methods described herein have shown that cells of distinct types can be unmixed purely *in silico*, provided that large numbers of single-cell expression profiles are generated.

Importantly, then, a very high-throughput, scalable method for single-cell expression profiling is required. Therefore, a method was developed to prepare a barcoded single-cell cDNA sample from 96 cells in a single incubation step. As a consequence, 96 cells could be pooled and treated as a single sample throughout the procedure, which greatly increased throughput and reduced cost. It can also reduce amplification bias, since all 96 cells were amplified in a single closed tube. The entire procedure takes two days to perform, from 96 living cells to finished samples loaded on the Genome Analyzer. The cost, including all reagents and consumables to generate 10-15 million 36 bp reads using commercial services, was approximately $3500 (that is, about $35/cell).

The date generated herein was on a large number of single cells, each analyzed at a relatively shallow depth of coverage. This allowed the generation of data on far more single cells than have ever been reported in a single study (no single-cell transcriptome experiments with more than a dozen cells have been published), and to produce a cell map with high resolution. In fact, provided that each cell is sampled deeply enough to cluster correctly, it would often make more sense to analyze a larger number of cells than to analyze each cell more deeply. The more cells are added, the more accurate will be the aggregate data obtained from each distinct cell type (cluster), and the better the resolution in "cell ype space". For example, many of the ES cells here were sampled at less than 100,000 reads/cell, but altogether 160 ES cells were identified in the cell map, comprising over 1.5 million reads. Sampling a large number of cells will be especially important when the approach is applied to complex tissues, where some types of cells can be present only in a small minority. In addition, as sequencing costs continue to decrease, the tradeoff between number of cells and number of reads will become less pressing.

The use of very large-scale single-cell transcriptional profiling to build a detailed map of naturally occurring cell types is envisioned, which would give unprecedented access to the genetic machinery active in each type of cell at each stage of development. The same strategy can be used to dissect the mutational heterogeneity of neoplasms at the single-cell level.

## Claims

1. A method of preparing a cDNA library from a plurality of single cells, the method comprising the steps of:
(i) releasing mRNA from each single cell to provide a plurality of individual mRNA samples, wherein the mRNA in each individual mRNA sample is from a single cell;
(ii) synthesizing a first strand of cDNA from the mRNA in each individual mRNA sample and incorporating a distinct tag or a distinct combination of tags into each individual cDNA sample to provide a plurality of tagged cDNA samples, wherein each cDNA sample has a distinct tag or combination of tags, wherein the cDNA in each tagged cDNA sample is complementary to mRNA from a single cell;
(iii)pooling the tagged cDNA samples; and
(iv)amplifying the pooled cDNA samples to generate a cDNA library comprising doublestranded cDNA.

2. The method according to claim 1, wherein in step (ii) the tag is incorporated into the cDNA during its synthesis.

3. The method according to claim 1 or 2, wherein synthesis of the first strand of cDNA in step (ii) is directed by a cDNA synthesis primer (CDS) that includes an RNA complementary sequence (RCS) that is at least partially complementary to one or more mRNA in an individual mRNA sample.

4. The method according to any one of claims 1-3, wherein the RCS is at least partially complementary to a portion of the first strand of cDNA, such that it is able to direct the synthesis of a second strand of cDNA using the first strand of cDNA as a template, or
wherein a template-switching oligonucleotide (TSO) is added to each individual mRNA sample, wherein said TSO comprises a portion which is at least partially complementary to a portion at the 3' end of the first strand of cDNA, wherein optionally the CDS or the TSO includes a tag, wherein preferably the tag is a nucleotide sequence of 4-20 nucleotides in length, or
wherein optionally both the CDS and the TSO include a tag, wherein preferably the CDS and the TSO each include a different tag, such that the tagged cDNA sample comprises a combination of tags.

5. The method according to any one of claims 1-4, wherein the first strand of cDNA includes a 3' portion comprising a plurality of cytosines or cytosine analogues that base pair with guanosine, wherein optionally the TSO includes a 3' portion comprising a plurality of guanosines or guanosine analogues that base pair with cytosine, wherein preferably the guanosines are ribonucleosides or locked nucleic acid monomers.

6. The method according to any one of claims 3-5, wherein the CDS comprises a 5' amplification primer sequence (APS) and a 3' RCS.

7. The method according to claim 6, wherein the 3' RCS comprises oligo(dT), a gene family-specific sequence, a random sequence or a non-self-complementary semi-random sequence, and/or wherein the TSO includes a 5' APS, wherein optionally the CDS and the 5' APS of the TSO is at least 80% identical to the 5' APS of the CDS, or wherein the CDS and the 5' APS of the TSO is 100% identical to the 5' APS of the CDS.

8. The method according to any one of claims 1-7, wherein the cells are lysed to release mRNA, and/or wherein the mRNA is purified following step (i), and/or wherein the synthesis of cDNA from mRNA is stopped before the tagged cDNA samples are pooled, and/or wherein the tagged cDNA samples are purified before amplification of the cDNA.

9. The method according to claim 1, wherein in step (iv) the pooled cDNA samples are amplified by PCR, wherein optionally the pooled cDNA samples are amplified by emulsion PCR, wherein preferably the pooled cDNA samples are amplified by single primer PCR.

10. The method according to any one of claims 1-9, wherein the method further comprises processing the cDNA library to obtain a library suitable for sequencing.

11. The method according to claim 10, wherein the processing comprises fragmenting the cDNA library and/or wherein the processing includes the step of adding an adapter to the cDNA, and/or wherein the cDNA library is amplified.

12. A cDNA library produced by the method of any one of claims 1 to 11.

13. A method for analysing gene expression in a plurality of single cells, the method comprising the steps of:
(i) preparing a cDNA library according to the method of any one of claims 1 to 11; and
(ii) sequencing the cDNA library.

14. The method according to claim 13, wherein sequencing is by shotgun sequencing, wherein optionally the cDNA library is sequenced to obtain at least 10,000, at least 1 million, at least 10 million, at least 100 million, or at least 1 billion reads, wherein a read is a length of continuous nucleic acid obtained by a sequencing reaction.

## Patentansprüche

1. Verfahren zur Herstellung einer cDNA-Bibliothek aus einer Vielzahl an Einzelzellen, wobei das Verfahren die Schritte umfasst:
i) Freisetzen von mRNA aus jeder einzelnen Zelle, um eine Vielzahl an individuellen mRNA-Proben bereitzustellen, wobei die mRNA in jeder individuellen mRNA-Probe aus einer Einzelzelle stammt;
ii) Synthetisieren eines ersten Stranges cDNA aus der mRNA in jeder individuellen mRNA-Probe und Einbauen einer eindeutigen Markierung oder einer eindeutigen Kombination von Markierungen in jede individuellen cDNA-Probe, um eine Vielzahl an markierten cDNA-Proben bereitzustellen, wobei jede cDNA-Probe eine eindeutige Markierung oder Kombination von Markierungen hat, wobei die cDNA in jeder markierten cDNA-Probe komplementär zur mRNA einer Einzelzelle ist;
iii) Vereinigen der markierten cDNA-Proben; und
iv) Amplifizieren der vereinigten cDNA-Proben, um eine cDNA-Bibliothek, die doppelsträngige cDNA aufweist, zu erzeugen.

2. Verfahren nach Anspruch 1, wobei in Schritt (ii) die Markierung in die cDNA während ihrer Synthese eingebaut wird.

3. Verfahren nach Anspruch 1 oder 2, wobei die Synthese des ersten Strangs der cDNA in Schritt (ii) von einem cDNA-Synthese-Primer (CDS) gelenkt wird, der eine zu RNA komplementäre Sequenz (RCS) enthält, die mindestens teilweise komplementär zu einer oder mehreren mRNA in einer individuellen mRNA-Probe ist.

4. Verfahren nach einem der Ansprüche 1-3, wobei die RCS mindestens teilweise komplementär zu einem Teil des ersten Stranges der cDNA ist, so dass sie in der Lage ist, die Synthese eines zweiten cDNA-Stranges unter Verwendung des ersten cDNA-Stranges als Matrize zu lenken, oder
wobei zu jeder individuellen mRNA-Probe ein Template-Switching-Oligonukleotid (TSO) hinzugefügt wird, wobei das TSO einen Teil aufweist, der mindestens teilweise komplementär zu einem Bereich am 3'-Ende des ersten Stranges der cDNA ist, wobei gegebenenfalls der CDS oder TSO eine Markierung aufweist, wobei vorzugsweise die Markierung eine Nukleotidsequenz von 4-20 Nukleotiden Länge ist, oder
wobei gegebenenfalls sowohl der CDS als auch das TSO eine Markierung aufweisen, wobei vorzugsweise der CDS und das TSO jeweils eine unterschiedliche Markierung aufweisen, so dass die markierte cDNA-Probe eine Kombination von Markierungen aufweist.

5. Verfahren nach einem der Ansprüche 1-4, wobei der erste Strang der cDNA einen 3'-Bereich aufweist, der eine Vielzahl an Cytosinen oder Cytosin-Analoga aufweist, die mit Guanosin ein Basenpaar bilden, wobei gegebenenfalls das TSO einen 3'-Bereich aufweist, der eine Vielzahl an Guanosinen oder Guanosin-Analoga aufweist, die mit Cytosin ein Basenpaar bilden, wobei vorzugsweise die Guanosine Ribonukleoside oder gesperrte Nukleinsäure-Monomere sind.

6. Verfahren nach einem der Ansprüche 3-5, wobei der CDS eine 5'-Amplifikationsprimer-Sequenz (APS) und eine 3'-RCS aufweist.

7. Verfahren nach Anspruch 6, wobei die 3'-RCS Oligo-(dT), eine Genfamilien-spezifische Sequenz, eine Zufallssequenz oder eine nicht-selbstkomplementäre halb-zufällige Sequenz aufweist, und/oder wobei das TSO eine 5'-APS enthält, wobei gegebenenfalls der CDS und die 5'-APS des TSO mindestens 80% identisch mit der 5'-APS des CDS sind, oder wobei der CDS und die 5'-APS des TSO 100% identisch mit den 5'-APS des CDS sind.

8. Verfahren nach einem der Ansprüche 1-7, wobei die Zellen lysiert werden, um mRNA freizusetzen, und/oder wobei die mRNA nach Schritt (i) aufgereinigt wird, und/oder wobei die Synthese von cDNA aus mRNA gestoppt wird, bevor die markierten cDNA-Proben vereinigt werden, und/oder wobei die markierten cDNA-Proben vor der Amplifikation der cDNA aufgereinigt werden.

9. Verfahren nach Anspruch 1, wobei in Schritt (iv) die vereinigten cDNA-Proben durch PCR amplifiziert werden, wobei gegebenenfalls die vereinigten cDNA-Proben durch Emulsions-PCR amplifiziert werden, wobei vorzugsweise die vereinigten cDNA-Proben durch Einzelprimer-PCR amplifiziert werden.

10. Verfahren nach einem der Ansprüche 1-9, wobei das Verfahren weiterhin das Verarbeiten der cDNA-Bibliothek umfasst, um eine für die Sequenzierung geeignete Bibliothek zu erhalten.

11. Verfahren nach Anspruch 10, wobei das Verarbeiten das Fragmentieren der cDNA-Bibliothek umfasst und/oder wobei das Verarbeiten den Schritt der Zugabe eines Adapters zu der cDNA umfasst, und/oder wobei die cDNA-Bibliothek amplifiziert wird.

12. cDNA-Bibliothek, die durch das Verfahren nach einem der Ansprüche 1 bis 11 hergestellt wird.

13. Verfahren zur Analyse der Genexpression in einer Vielzahl an Einzelzellen, wobei das Verfahren die Schritte umfasst:
i) Herstellen einer cDNA-Bibliothek nach dem Verfahren nach einem der Ansprüche 1 bis 11; und
ii) Sequenzieren der cDNA-Bibliothek.

14. Verfahren nach Anspruch 13, wobei Sequenzieren Shotgun-Sequenzieren ist, wobei gegebenenfalls die cDNA-Bibliothek sequenziert wird, um mindestens 10.000, mindestens 1 Million, mindestens 10 Millionen, mindestens 100 Millionen oder mindestens 1 Milliarde Lesungen zu erhalten, wobei eine Lesung eine Länge an kontinuierlicher Nukleinsäure ist, die durch eine Sequenzierungsreaktion erhalten wird.

## Revendications

1. Procédé de préparation d'une banque d'ADNc à partir d'une pluralité de cellules uniques, le procédé comprenant les étapes de :
(i) libération de l'ARNm de chaque cellule unique pour fournir une pluralité d'échantillons d'ARNm individuels, l'ARNm de chaque échantillon d'ARNm individuel provenant d'une cellule unique ;
(ii) synthèse d'un premier brin d'ADNc à partir de l'ARNm dans chaque échantillon d'ARNm individuel, et incorporation d'une étiquette distincte ou d'une combinaison distincte d'étiquettes dans chaque échantillon d'ADNc individuel pour fournir une pluralité d'échantillons d'ADNc étiquetés, chaque échantillon d'ADNc ayant une étiquette distincte ou une combinaison distincte d'étiquettes, l'ADNc se trouvant dans chaque échantillon d'ADNc étiqueté étant complémentaire de l'ARNm provenant d'une cellule unique ;
(iii) regroupement des échantillons d'ADNc étiquetés ; et
(iv) amplification des échantillons d'ADNc regroupés pour générer une banque d'ADNc comprenant des ADNc double brin.

2. Procédé selon la revendication 1, dans lequel, dans l'étape (ii), l'étiquette est incorporée dans l'ADNc pendant sa synthèse.

3. Procédé selon la revendication 1 ou 2, dans lequel la synthèse du premier brin de l'ADNc dans l'étape (ii) est dirigée par une amorce de synthèse d'ADNc (CDS) qui comprend une séquence complémentaire d'ARN (RCS) qui est au moins partiellement complémentaire d'un ou plusieurs ARNm d'un échantillon d'ARNm individuel.

4. Procédé selon l'une quelconque des revendications 1 à 3, dans lequel le RCS est au moins partiellement complémentaire d'une portion du premier brin de l'ADNc de telle sorte qu'il soit capable de diriger la synthèse d'un deuxième brin de l'ADNc par utilisation en tant que matrice du premier brin de l'ADNc, ou
dans lequel un oligonucléotide de changement de matrice (TSO) est ajouté à chaque échantillon d'ARNm individuel, ledit TSO comprenant une portion qui est au moins partiellement complémentaire d'une portion au niveau de l'extrémité 3' du premier brin de l'ADNc, en option le CDS ou le TSO comprenant une étiquette, l'étiquette étant de préférence une séquence nucléotidique ayant une longueur de 4 à 20 nucléotides, ou
dans lequel en option tant le CDS que le TSO comprend une étiquette, de préférence le CDS et le TSO comprenant chacun une étiquette différente, de telle sorte que l'échantillon d'ADNc étiqueté comprenne une combinaison d'étiquettes.

5. Procédé selon l'une quelconque des revendications 1 à 4, dans lequel le premier brin de l'ADNc comprend une portion en 3' comprenant une pluralité de cytosines ou d'analogues de cytosines, qui s'apparient par appariement des bases avec la guanosine, en option le TSO comprenant une portion en 3' comprenant une pluralité de guanosines ou d'analogues de guanosine qui s'apparient par appariement des bases avec une cytosine, de préférence les cytosines étant des ribonucléosides ou des acides nucléiques monomères verrouillés.

6. Procédé selon l'une quelconque des revendications 3 à 5, dans lequel le CDS comprend une séquence d'amorce pour amplification en 5' (APS) et un 3'-RCS.

7. Procédé selon la revendication 6, dans lequel le 3'-RCS comprend un oligo(dT), une séquence spécifique de famille de gènes, une séquence aléatoire ou une séquence semi-aléatoire non-autocomplémentaire, et/ou dans lequel le TSO comprend un 5'-APS, en option le CDS et le 5'-APS du TSO ayant une identité d'au moins 80 % avec le 5'-APS du CDS, ou le CDS et le 5'-APS du TSO ayant une identité de 100 % avec le 5'-APS du CDS.

8. Procédé selon l'une quelconque des revendications 1 à 7, dans lequel les cellules sont lysées pour libérer l'ARNm, et/ou dans lequel l'ARNm est purifié après l'étape (i), et/ou dans lequel la synthèse de l'ADNc à partir de l'ARNm est interrompue avant regroupement des échantillons d'ADNc étiquetés, et/ou dans lequel les échantillons d'ADNc étiquetés sont purifiés avant amplification de l'ADNc.

9. Procédé selon la revendication 1, dans lequel, dans l'étape (iv), les échantillons d'ADNc regroupés sont amplifiés par PCR, les échantillons d'ADNc regroupés étant amplifiés par PCR en émulsion, de préférence les échantillons d'ADNc regroupés étant amplifiés par PCR à amorce unique.

10. Procédé selon l'une quelconque des revendications 1 à 9, le procédé comprenant le traitement de la banque d'ADNc pour obtenir une banque convenant au séquençage.

11. Procédé selon la revendication 10, dans lequel le traitement comprend la fragment de la banque d'ADNc, et/ou le traitement comprenant l'étape d'addition d'un adaptateur à l'ADNc, et/ou dans lequel la banque d'ADNc est amplifiée.

12. Banque d'ADNc produite par le procédé selon l'une quelconque des revendications 1 à 11.

13. Procédé pour l'analyse de l'expression génique dans une pluralité de cellules uniques, le procédé comprenant les étapes de :
(i) préparation d'une banque d'ADNc par le procédé selon l'une quelconque des revendications 1 à 11 ; et
(ii) séquençage de la banque d'ADNc.

14. Procédé selon la revendication 13, dans lequel le séquençage s'effectue par séquençage en aveugle, dans lequel la banque d'ADNc est en option séquencée pour obtenir au moins 10 000, au moins 1 million, au moins 10 millions, au moins 100 millions ou au moins 1 milliard de lectures, une lecture étant une longueur d'un acide nucléique continu obtenu par une réaction de séquençage.


### SEQUENCE LISTING

<110> Illumina, Inc.
<120> Gene Expression Analysis in Single Cells
<130> IP-0476-US
<150> 61/164,759
   <151> 2009-03-30
<150> PCT/US2010/028361
   <151> 2010-03-23
<160> 111
<170> PatentIn version 3.5
<210> 1
   <211> 23
   <212> DNA
   <213> Artificial Sequence
<220>
   <223> STRT-T30-BIO Primer
<400> 1
   aagcagtggt atcaacgcag agt 23
<210> 2
   <211> 38
   <212> DNA
   <213> Artificial Sequence
<220>
   <223> STRT-FW-n
<220>
   <221> misc_feature
   <222> (31) .. (35)
   <223> n is a, c, g, or t
<400> 2
   aagcagtggt atcaacgcag agtggatgct nnnnnggg 38
<210> 3
   <211> 23
   <212> DNA
   <213> Artificial Sequence
<220>
   <223> STRT-PCR primer
<400> 3
   aagcagtggt atcaacgcag agt 23
<210> 4
   <211> 43
   <212> DNA
   <213> Artificial Sequence
<220>
   <223> STRT-ADP1U
<400> 4
   ccactacgcc tccgctttcc tctctatggg cagtcggtga tct 43
<210> 5
   <211> 41
   <212> DNA
   <213> Artificial Sequence
<220>
   <223> STRT-ADP1L
<400> 5
   ggtgatgcgg aggcgaaagg agagataccc gtcagccact a 41
<210> 6
   <211> 26
   <212> DNA
   <213> Artificial Sequence
<220>
   <223> STRT-ADP2U-T
<400> 6
   aactgccccg ggttcctcat tctctt 26
<210> 7
   <211> 25
   <212> DNA
   <213> Artificial Sequence
<220>
   <223> STRT-ADP2L
<400> 7
   ttgacggggc ccaaggagta agaga 25
<210> 8
   <211> 28
   <212> DNA
   <213> Artificial Sequence
<220>
   <223> SOLID-P1
<400> 8
   ccactacgcc tccgctttcc tctctatg 28
<210> 9
   <211> 23
   <212> DNA
   <213> Artificial Sequence
<220>
   <223> SOLID-P2
<400> 9
   ctgccccggg ttcctcattc tct 23
<210> 10
   <211> 38
   <212> DNA
   <213> Artificial Sequence
<220>
   <223> STRT-FW-1
<400> 10
   aagcagtggt atcaacgcag agtggatgct cagaaggg 38
<210> 11
   <211> 38
   <212> DNA
   <213> Artificial Sequence
<220>
   <223> STRT-FW-2
<400> 11
   aagcagtggt atcaacgcag agtggatgct catacggg 38
<210> 12
   <211> 38
   <212> DNA
   <213> Artificial Sequence
<220>
   <223> STRT-FW-3
<400> 12
   aagcagtggt atcaacgcag agtggatgct caaagggg 38
<210> 13
   <211> 38
   <212> DNA
   <213> Artificial Sequence
<220>
   <223> STRT-FW-4
<400> 13
   aagcagtggt atcaacgcag agtggatgct cacatggg 38
<210> 14
   <211> 38
   <212> DNA
   <213> Artificial Sequence
<220>
   <223> STRT-FW-5
<400> 14
   aagcagtggt atcaacgcag agtggatgct catcaggg 38
<210> 15
   <211> 38
   <212> DNA
   <213> Artificial Sequence
<220>
   <223> STRT-FW-6
<400> 15
   aagcagtggt atcaacgcag agtggatgct cagccggg 38
<210> 16
   <211> 38
   <212> DNA
   <213> Artificial Sequence
<220>
   <223> STRT-FW-7
<400> 16
   aagcagtggt atcaacgcag agtggatgct caccgggg 38
<210> 17
   <211> 38
   <212> DNA
   <213> Artificial Sequence
<220>
   <223> STRT-FW-8
<400> 17
   aagcagtggt atcaacgcag agtggatgct caactggg 38
<210> 18
   <211> 38
   <212> DNA
   <213> Artificial Sequence
<220>
   <223> STRT-FW-9
<400> 18
   aagcagtggt atcaacgcag agtggatgct caagaggg 38
<210> 19
   <211> 38
   <212> DNA
   <213> Artificial Sequence
<220>
   <223> STRT-FW-10
<400> 19
   aagcagtggt atcaacgcag agtggatgct cacgcggg 38
<210> 20
   <211> 38
   <212> DNA
   <213> Artificial Sequence
<220>
   <223> STRT-FW-11
<400> 20
   aagcagtggt atcaacgcag agtggatgct catgtggg 38
<210> 21
   <211> 38
   <212> DNA
   <213> Artificial Sequence
<220>
   <223> STRT-FW-12
<400> 21
   aagcagtggt atcaacgcag agtggatgct cactaggg 38
<210> 22
   <211> 38
   <212> DNA
   <213> Artificial Sequence
<220>
   <223> STRT-FW-13
<400> 22
   aagcagtggt atcaacgcag agtggatgct caatcggg 38
<210> 23
   <211> 38
   <212> DNA
   <213> Artificial Sequence
<220>
   <223> STRT-FW-14
<400> 23
   aagcagtggt atcaacgcag agtggatgct cattgggg 38
<210> 24
   <211> 38
   <212> DNA
   <213> Artificial Sequence
<220>
   <223> STRT-FW-15
<400> 24
   aagcagtggt atcaacgcag agtggatgct cagttggg 38
<210> 25
   <211> 38
   <212> DNA
   <213> Artificial Sequence
<220>
   <223> STRT-FW-16
<400> 25
   aagcagtggt atcaacgcag agtggatgct cctaaggg 38
<210> 26
   <211> 38
   <212> DNA
   <213> Artificial Sequence
<220>
   <223> STRT-FW-17
<400> 26
   aagcagtggt atcaacgcag agtggatgct ccgacggg 38
<210> 27
   <211> 38
   <212> DNA
   <213> Artificial Sequence
<220>
   <223> STRT-FW-18
<400> 27
   aagcagtggt atcaacgcag agtggatgct ccaatggg 38
<210> 28
   <211> 38
   <212> DNA
   <213> Artificial Sequence
<220>
   <223> STRT-FW-19
<400> 28
   aagcagtggt atcaacgcag agtggatgct ccgcaggg 38
<210> 29
   <211> 38
   <212> DNA
   <213> Artificial Sequence
<220>
   <223> STRT-FW-20
<400> 29
   aagcagtggt atcaacgcag agtggatgct cctccggg 38
<210> 30
   <211> 38
   <212> DNA
   <213> Artificial Sequence
<220>
   <223> STRT-FW-21
<400> 30
   aagcagtggt atcaacgcag agtggatgct ccacgggg 38
<210> 31
   <211> 38
   <212> DNA
   <213> Artificial Sequence
<220>
   <223> STRT-FW-22
<400> 31
   aagcagtggt atcaacgcag agtggatgct ccagcggg 38
<210> 32
   <211> 38
   <212> DNA
   <213> Artificial Sequence
<220>
   <223> STRT-FW-23
<400> 32
   aagcagtggt atcaacgcag agtggatgct cctggggg 38
<210> 33
   <211> 38
   <212> DNA
   <213> Artificial Sequence
<220>
   <223> STRT-FW-24
<400> 33
   aagcagtggt atcaacgcag agtggatgct ccggtggg 38
<210> 34
   <211> 38
   <212> DNA
   <213> Artificial Sequence
<220>
   <223> STRT-FW-25
<400> 34
   aagcagtggt atcaacgcag agtggatgct ccataggg 38
<210> 35
   <211> 38
   <212> DNA
   <213> Artificial Sequence
<220>
   <223> STRT-FW-26
<400> 35
   aagcagtggt atcaacgcag agtggatgct ccgtgggg 38
<210> 36
   <211> 38
   <212> DNA
   <213> Artificial Sequence
<220>
   <223> STRT-FW-27
<400> 36
   aagcagtggt atcaacgcag agtggatgct cctttggg 38
<210> 37
   <211> 38
   <212> DNA
   <213> Artificial Sequence
<220>
   <223> STRT-FW-28
<400> 37
   aagcagtggt atcaacgcag agtggatgct cgaaaggg 38
<210> 38
   <211> 38
   <212> DNA
   <213> Artificial Sequence
<220>
   <223> STRT-FW-29
<400> 38
   aagcagtggt atcaacgcag agtggatgct cgcacggg 38
<210> 39
   <211> 38
   <212> DNA
   <213> Artificial Sequence
<220>
   <223> STRT-FW-30
<400> 39
   aagcagtggt atcaacgcag agtggatgct cggagggg 38
<210> 40
   <211> 38
   <212> DNA
   <213> Artificial Sequence
<220>
   <223> STRT-FW-31
<400> 40
   aagcagtggt atcaacgcag agtggatgct cgtatggg 38
<210> 41
   <211> 38
   <212> DNA
   <213> Artificial Sequence
<220>
   <223> STRT-FW-32
<400> 41
   aagcagtggt atcaacgcag agtggatgct cgccaggg 38
<210> 42
   <211> 38
   <212> DNA
   <213> Artificial Sequence
<220>
   <223> STRT-FW-33
<400> 42
   aagcagtggt atcaacgcag agtggatgct cgaccggg 38
<210> 43
   <211> 38
   <212> DNA
   <213> Artificial Sequence
<220>
   <223> STRT-FW-34
<400> 43
   aagcagtggt atcaacgcag agtggatgct cgtcgggg 38
<210> 44
   <211> 38
   <212> DNA
   <213> Artificial Sequence
<220>
   <223> STRT-FW-35
<400> 44
   aagcagtggt atcaacgcag agtggatgct cggctggg 38
<210> 45
   <211> 38
   <212> DNA
   <213> Artificial Sequence
<220>
   <223> STRT-FW-36
<400> 45
   aagcagtggt atcaacgcag agtggatgct cgggaggg 38
<210> 46
   <211> 38
   <212> DNA
   <213> Artificial Sequence
<220>
   <223> STRT-FW-37
<400> 46
   aagcagtggt atcaacgcag agtggatgct cgtgcggg 38
<210> 47
   <211> 38
   <212> DNA
   <213> Artificial Sequence
<220>
   <223> STRT-FW-38
<400> 47
   aagcagtggt atcaacgcag agtggatgct cgaggggg 38
<210> 48
   <211> 38
   <212> DNA
   <213> Artificial Sequence
<220>
   <223> STRT-FW-39
<400> 48
   aagcagtggt atcaacgcag agtggatgct cgcgtggg 38
<210> 49
   <211> 38
   <212> DNA
   <213> Artificial Sequence
<220>
   <223> STRT-FW-40
<400> 49
   aagcagtggt atcaacgcag agtggatgct cgttaggg 38
<210> 50
   <211> 38
   <212> DNA
   <213> Artificial Sequence
<220>
   <223> STRT-FW-41
<400> 50
   aagcagtggt atcaacgcag agtggatgct cggtcggg 38
<210> 51
   <211> 38
   <212> DNA
   <213> Artificial Sequence
<220>
   <223> STRT-FW-42
<400> 51
   aagcagtggt atcaacgcag agtggatgct cgctgggg 38
<210> 52
   <211> 38
   <212> DNA
   <213> Artificial Sequence
<220>
   <223> STRT-FW-43
<400> 52
   aagcagtggt atcaacgcag agtggatgct cgattggg 38
<210> 53
   <211> 38
   <212> DNA
   <213> Artificial Sequence
<220>
   <223> STRT-FW-44
<400> 53
   aagcagtggt atcaacgcag agtggatgct ctcaaggg 38
<210> 54
   <211> 38
   <212> DNA
   <213> Artificial Sequence
<220>
   <223> STRT-FW-45
<400> 54
   aagcagtggt atcaacgcag agtggatgct ctaacggg 38
<210> 55
   <211> 38
   <212> DNA
   <213> Artificial Sequence
<220>
   <223> STRT-FW-46
<400> 55
   aagcagtggt atcaacgcag agtggatgct cttagggg 38
<210> 56
   <211> 38
   <212> DNA
   <213> Artificial Sequence
<220>
   <223> STRT-FW-47
<400> 56
   aagcagtggt atcaacgcag agtggatgct ctgatggg 38
<210> 57
   <211> 38
   <212> DNA
   <213> Artificial Sequence
<220>
   <223> STRT-FW-48
<400> 57
   aagcagtggt atcaacgcag agtggatgct ctacaggg 38
<210> 58
   <211> 38
   <212> DNA
   <213> Artificial Sequence
<220>
   <223> STRT-FW-49
<400> 58
   aagcagtggt atcaacgcag agtggatgct ctgcgggg 38
<210> 59
   <211> 38
   <212> DNA
   <213> Artificial Sequence
<220>
   <223> STRT-FW-50
<400> 59
   aagcagtggt atcaacgcag agtggatgct cttctggg 38
<210> 60
   <211> 38
   <212> DNA
   <213> Artificial Sequence
<220>
   <223> STRT-FW-51
<400> 60
   aagcagtggt atcaacgcag agtggatgct cttgaggg 38
<210> 61
   <211> 38
   <212> DNA
   <213> Artificial Sequence
<220>
   <223> STRT-FW-52
<400> 61
   aagcagtggt atcaacgcag agtggatgct ctggcggg 38
<210> 62
   <211> 38
   <212> DNA
   <213> Artificial Sequence
<220>
   <223> STRT-FW-53
<400> 62
   aagcagtggt atcaacgcag agtggatgct ctcggggg 38
<210> 63
   <211> 38
   <212> DNA
   <213> Artificial Sequence
<220>
   <223> STRT-FW-54
<400> 63
   aagcagtggt atcaacgcag agtggatgct ctagtggg 38
<210> 64
   <211> 38
   <212> DNA
   <213> Artificial Sequence
<220>
   <223> STRT-FW-55
<400> 64
   aagcagtggt atcaacgcag agtggatgct ctgtaggg 38
<210> 65
   <211> 38
   <212> DNA
   <213> Artificial Sequence
<220>
   <223> STRT-FW-56
<400> 65
   aagcagtggt atcaacgcag agtggatgct ctttcggg 38
<210> 66
   <211> 38
   <212> DNA
   <213> Artificial Sequence
<220>
   <223> STRT-FW-57
<400> 66
   aagcagtggt atcaacgcag agtggatgct ctatgggg 38
<210> 67
   <211> 38
   <212> DNA
   <213> Artificial Sequence
<220>
   <223> STRT-FW-58
<400> 67
   aagcagtggt atcaacgcag agtggatgct ctcttggg 38
<210> 68
   <211> 38
   <212> DNA
   <213> Artificial Sequence
<220>
   <223> STRT-FW-59
<400> 68
   aagcagtggt atcaacgcag agtggatgct gacaaggg 38
<210> 69
   <211> 38
   <212> DNA
   <213> Artificial Sequence
<220>
   <223> STRT-FW-60
<400> 69
   aagcagtggt atcaacgcag agtggatgct gaaacggg 38
<210> 70
   <211> 38
   <212> DNA
   <213> Artificial Sequence
<220>
   <223> STRT-FW-61
<400> 70
   aagcagtggt atcaacgcag agtggatgct gatagggg 38
<210> 71
   <211> 38
   <212> DNA
   <213> Artificial Sequence
<220>
   <223> STRT-FW-62
<400> 71
   aagcagtggt atcaacgcag agtggatgct gagatggg 38
<210> 72
   <211> 38
   <212> DNA
   <213> Artificial Sequence
<220>
   <223> STRT-FW-63
<400> 72
   aagcagtggt atcaacgcag agtggatgct gaacaggg 38
<210> 73
   <211> 38
   <212> DNA
   <213> Artificial Sequence
<220>
   <223> STRT-FW-64
<400> 73
   aagcagtggt atcaacgcag agtggatgct gagcgggg 38
<210> 74
   <211> 38
   <212> DNA
   <213> Artificial Sequence
<220>
   <223> STRT-FW-65
<400> 74
   aagcagtggt atcaacgcag agtggatgct gatctggg 38
<210> 75
   <211> 38
   <212> DNA
   <213> Artificial Sequence
<220>
   <223> STRT-FW-66
<400> 75
   aagcagtggt atcaacgcag agtggatgct gatgaggg 38
<210> 76
   <211> 38
   <212> DNA
   <213> Artificial Sequence
<220>
   <223> STRT-FW-67
<400> 76
   aagcagtggt atcaacgcag agtggatgct gaggcggg 38
<210> 77
   <211> 38
   <212> DNA
   <213> Artificial Sequence
<220>
   <223> STRT-FW-68
<400> 77
   aagcagtggt atcaacgcag agtggatgct gacggggg 38
<210> 78
   <211> 38
   <212> DNA
   <213> Artificial Sequence
<220>
   <223> STRT-FW-69
<400> 78
   aagcagtggt atcaacgcag agtggatgct gaagtggg 38
<210> 79
   <211> 38
   <212> DNA
   <213> Artificial Sequence
<220>
   <223> STRT-FW-70
<400> 79
   aagcagtggt atcaacgcag agtggatgct gagtaggg 38
<210> 80
   <211> 38
   <212> DNA
   <213> Artificial Sequence
<220>
   <223> STRT-FW-71
<400> 80
   aagcagtggt atcaacgcag agtggatgct gattcggg 38
<210> 81
   <211> 38
   <212> DNA
   <213> Artificial Sequence
<220>
   <223> STRT-FW-72
<400> 81
   aagcagtggt atcaacgcag agtggatgct gaatgggg 38
<210> 82
   <211> 38
   <212> DNA
   <213> Artificial Sequence
<220>
   <223> STRT-FW-73
<400> 82
   aagcagtggt atcaacgcag agtggatgct gacttggg 38
<210> 83
   <211> 38
   <212> DNA
   <213> Artificial Sequence
<220>
   <223> STRT-FW-74
<400> 83
   aagcagtggt atcaacgcag agtggatgct gcaaaggg 38
<210> 84
   <211> 38
   <212> DNA
   <213> Artificial Sequence
<220>
   <223> STRT-FW-75
<400> 84
   aagcagtggt atcaacgcag agtggatgct gccacggg 38
<210> 85
   <211> 38
   <212> DNA
   <213> Artificial Sequence
<220>
   <223> STRT-FW-76
<400> 85
   aagcagtggt atcaacgcag agtggatgct gcgagggg 38
<210> 86
   <211> 38
   <212> DNA
   <213> Artificial Sequence
<220>
   <223> STRT-FW-77
<400> 86
   aagcagtggt atcaacgcag agtggatgct gctatggg 38
<210> 87
   <211> 38
   <212> DNA
   <213> Artificial Sequence
<220>
   <223> STRT-FW-78
<400> 87
   aagcagtggt atcaacgcag agtggatgct gcaccggg 38
<210> 88
   <211> 38
   <212> DNA
   <213> Artificial Sequence
<220>
   <223> STRT-FW-79
<400> 88
   aagcagtggt atcaacgcag agtggatgct gctcgggg 38
<210> 89
   <211> 38
   <212> DNA
   <213> Artificial Sequence
<220>
   <223> STRT-FW-80
<400> 89
   aagcagtggt atcaacgcag agtggatgct gcgctggg 38
<210> 90
   <211> 38
   <212> DNA
   <213> Artificial Sequence
<220>
   <223> STRT-FW-81
<400> 90
   aagcagtggt atcaacgcag agtggatgct gcggaggg 38
<210> 91
   <211> 38
   <212> DNA
   <213> Artificial Sequence
<220>
   <223> STRT-FW-82
<400> 91
   aagcagtggt atcaacgcag agtggatgct gctgcggg 38
<210> 92
   <211> 38
   <212> DNA
   <213> Artificial Sequence
<220>
   <223> STRT-FW-83
<400> 92
   aagcagtggt atcaacgcag agtggatgct gcaggggg 38
<210> 93
   <211> 38
   <212> DNA
   <213> Artificial Sequence
<220>
   <223> STRT-FW-84
<400> 93
   aagcagtggt atcaacgcag agtggatgct gccgtggg 38
<210> 94
   <211> 38
   <212> DNA
   <213> Artificial Sequence
<220>
   <223> STRT-FW-85
<400> 94
   aagcagtggt atcaacgcag agtggatgct gcttaggg 38
<210> 95
   <211> 38
   <212> DNA
   <213> Artificial Sequence
<220>
   <223> STRT-FW-86
<400> 95
   aagcagtggt atcaacgcag agtggatgct gcgtcggg 38
<210> 96
   <211> 38
   <212> DNA
   <213> Artificial Sequence
<220>
   <223> STRT-FW-87
<400> 96
   aagcagtggt atcaacgcag agtggatgct gcctgggg 38
<210> 97
   <211> 38
   <212> DNA
   <213> Artificial Sequence
<220>
   <223> STRT-FW-88
<400> 97
   aagcagtggt atcaacgcag agtggatgct gcattggg 38
<210> 98
   <211> 38
   <212> DNA
   <213> Artificial Sequence
<220>
   <223> STRT-FW-89
<400> 98
   aagcagtggt atcaacgcag agtggatgct ggtaaggg 38
<210> 99
   <211> 38
   <212> DNA
   <213> Artificial Sequence
<220>
   <223> STRT-FW-90
<400> 99
   aagcagtggt atcaacgcag agtggatgct ggcagggg 38
<210> 100
   <211> 38
   <212> DNA
   <213> Artificial Sequence
<220>
   <223> STRT-FW-91
<400> 100
   aagcagtggt atcaacgcag agtggatgct ggaatggg 38
<210> 101
   <211> 38
   <212> DNA
   <213> Artificial Sequence
<220>
   <223> STRT-FW-92
<400> 101
   aagcagtggt atcaacgcag agtggatgct ggtccggg 38
<210> 102
   <211> 38
   <212> DNA
   <213> Artificial Sequence
<220>
   <223> STRT-FW-93
<400> 102
   aagcagtggt atcaacgcag agtggatgct ggacgggg 38
<210> 103
   <211> 38
   <212> DNA
   <213> Artificial Sequence
<220>
   <223> STRT-FW-94
<400> 103
   aagcagtggt atcaacgcag agtggatgct ggcctggg 38
<210> 104
   <211> 38
   <212> DNA
   <213> Artificial Sequence
<220>
   <223> STRT-FW-95
<400> 104
   aagcagtggt atcaacgcag agtggatgct ggcgaggg 38
<210> 105
   <211> 38
   <212> DNA
   <213> Artificial Sequence
<220>
   <223> STRT-FW-96
<400> 105
   aagcagtggt atcaacgcag agtggatgct ggagcggg 38
<210> 106
   <211> 58
   <212> DNA
   <213> Artificial Sequence
<220>
   <223> ADP1 Adapter Strand
<400> 106
   aatgatacgg cgaccaccga gatctacact ctttccctac acgacgctct tccgatct 58
<210> 107
   <211> 56
   <212> DNA
   <213> Artificial Sequence
<220>
   <223> ADP1 Adapter Strand 2
<400> 107
   ttactatgcc gctggtggct ctagatgtga gaaagggatg tgctgcgaga aggcta 56
<210> 108
   <211> 34
   <212> DNA
   <213> Artificial Sequence
<220>
   <223> ADP2 Adapter Strand
<400> 108
   caagcagaag acggcatacg agctcttccg atct 34
<210> 109
   <211> 33
   <212> DNA
   <213> Artificial Sequence
<220>
   <223> ADP2 Adapter Strand 2
<400> 109
   gttcgtcttc tgccgtatgc tcgagaaggc tag 33
<210> 110
   <211> 20
   <212> DNA
   <213> Artificial Sequence
<220>
   <223> Primer 1
<400> 110
   aatgatacgg cgaccaccga 20
<210> 111
   <211> 22
   <212> DNA
   <213> Artificial Sequence
<220>
   <223> Primer 2
<400> 111
   caagcagaag acggcatacg ag 22